# EUROPEAN PATENT APPLICATION

(11) **EP 2 787 349 A1**
(43) Date of publication of application: **08.10.2014**
(21) Application number: 13162109.6
(22) Date of filing: 03.04.2013
(51) Int. Cl.: G01N 33/564, C07K 14/47

(54) **Method for detecting proteinopathy-specific antibodies in a biological sample**

(71) Applicant: Affiris AG, 1030 Wien (AT)
(72) Inventor: Mandler, Markus, 1030 Vienna (AT); Staffler, Günther, 1030 Vienna (AT); Santic, Radmila, 1030 Vienna (AT); Mairhofer, Andreas, 1030 Vienna (AT)
(74) Representative: Sonn & Partner Patentanwälte

(57) **Abstract**

Disclosed is a method for detecting proteinopathy-specific antibodies in a biological sample comprising the following steps:
- contacting the sample with aggregates, comprising two or more of a protein species selected from the group consisting of alpha synuclein (aSyn) or an aggregate-forming variant thereof, Tau protein or an aggregate-forming variant thereof, amyloid beta (Aβ)1-42 peptide or an aggregate-forming variant thereof; or contacting the sample with particles having aggregate like surfaces comprising two or more of said protein species, and allowing the proteinopathy-specific antibodies to bind to the aggregates, and
- detecting the proteinopathy-specific antibodies bound to the aggregates by a single particle detection technique, preferably by fluorescence activated cell sorting (FACS).

## Description

The present invention relates to methods for detecting proteinopathy-specific antibodies in biological samples.

Proteinopathies are diseases, especially neurodegenerative diseases that are caused by or connected to a malformed protein. Various degenerative diseases are characterized by the aberrant polymerization and accumulation of specific, pathologically relevant proteins. These so called proteopathies include neurological disorders (ND) such as Alzheimer's disease (AD), Parkinson's disease (PD) as well as diverse systemic and neurodegenerative disorders including the amyloidoses, synucleinopathies, tauopathies, amyotrophic lateral sclerosis (ALS) and type II diabetes (T2D).

In proteopathies usually a change in its conformation increases the tendency of a specific, patho-physiologically relevant protein to aggregate, form soluble oligomeric and subsequently insoluble, mostly fibrillar aggregates which are deposited and form the typical pathological hallmarks of the specific proteopathies. Examples for such deposits are senile, amyloid plaques and neurofibrillary tangles in AD, mainly consisting of the amyloid beta peptides Aβ1-40/42 and hyperphosphorylated Tau, or Lewy Bodies and Glial cytoplasmic inclusions in Parkinson's disease and Multiple System atrophy, respectively.

Importantly, the formation of such potentially toxic aggregates (oligomeric and/or fibrillar) is usually involving different forms of the respective proteins as well as coaggregation of other potentially disease causing molecules.

One of the most important examples for such aggregations are associated with amyloid plaques in human AD-brain. Proteomic and histochemical analyses of such amyloid deposits revealed the presence of more than only amyloid ß peptide (i.e: Aβ1-40 and Aβ1-42, truncated Aβ as well as modified Aβ including the highly toxic pyro-glutamate modified forms Aβ(pE)3-40/42 and Aβ(pE)11-40/42). Several research groups demonstrated the coaggregation of Aβ with the disease related proteins ubiquitin, alpha Synuclein (typically found in LBs or GCIs in PD and MSA), and hyperphosphorylated Tau (usually found in NFTs (AD), Pick bodies (Pick's disease) and other Tauopathy lesions). Alpha Synuclein was even isolated for the first time from amyloid plaques and later associated with the lesions present in synucleinopathies. In addition further proteins not directly associated with neurodegeneration can also be detected including GFAP, Vimentin or neurofilaments.

LBs do not only contain alpha Synuclein but also show coaggregated hyperphosphorylated Tau, ubiquitin, neurofilament protein, and alpha B crystallin. Similarly, alpha Synuclein proteins can also be detected in Huntington's disease inclusion bodies, where they are coaggreagted with mutant HTT protein.

Mixed pathologic lesions of Aβ, hyperphosphorylated Tau and aSyn can be readily detected in various amyloidoses, tauopathies and synucleinopathies including Alzheimer's disease (AD), Parkinson's disease (PD), Dementia with Lewy Bodies (DLB), Frontotemporal Dementia/Pick's Disease (FTD/PiD) or Progressive Supranuclear Palsy (PSP).

Coaggregation of potentially toxic molecules is not only a pathologic hallmark of degenerative proteopathies including AD DLB, PD/LBD, Parkinson's Disease Dementia (PDD) and FTD as well as PSP or Corticobasal degeneration (CBD) but these coaggregations are also associated with synergistic pathologic activities. It has been shown by different research groups that co-expression and aggregation of Aβ and aSyn and/or Tau in animal models is exacerbating the pathologic activities of the single molecules in these models. In the case of AD it has been demonstrated by Nussbaum et al. (Nature. 2012 May 2; 485(7400): 651-655.) that Aβ aggregation and toxicity is enhanced by the coaggregation of full length and modified Aβ (Aβ(pE)3-42). In addition these authors could also demonstrate that the modified Aβ moiety involved is also crucially involved in eliciting the Tau dependent neurotoxicity in animal models of AD. Oddo et al.(Neuron. 2004 Aug 5;43(3):321-32.) could show earlier that immunotherapeutic reduction of Aβ could indeed also reduce Tau hyperphosphorylation and its associated pathology, supporting the notion of a tight interplay of these molecules in neuropathologic processes.

The present invention relates to methods for detecting specific antibodies in biological samples directed against different pathologically relevant molecules present in proteopathies, especially in connection with and for diagnosing of proteinopathies, preferably β-Amyloidoses, synucleinopathies, Tauopathies, especially ALS and T2D.

Examples for proteinopathies are synucleinopathies, including Parkinson's disease (PD), Lewy Body Disease (LBD), Dementia with Lewy Bodies (DLB), Parkinson's Disease Dementia (PDD), Multiple System Atrophy (MSA), Pure Autonomic Failure (PAF), REM Sleep Behavior Disorder (RBD), Neurodegeneration with Brain Iron Accumulation type I (NBIA Type I) and inclusion body myositis (IBM); and diseases with protein deposition and/or aggregation, including Alzheimer's disease (AD), Down Syndrome (DS), Progressive Supranuclear Palsy (PSP), Corticobasal degeneration (CBD), Frontotemporal Dementia/Pick's Disease (FTD/PiD), ALS and T2D.

Detection of antibodies that are characteristic for proteinopathies, especially autoantibodies against the malformed protein connected to the disease is difficult; therefore the diagnostic value of such determination, especially in patient's blood, has not been fully considered and understood.

There is also a lack of a reliable biomarker that can be applied repetitively, at low risk and costs. This is especially true since all efforts taken so far to develop blood-based biomarkers for proteinopathies, especially AD, PD and MSA, failed (Hampel et al., Nat. Rev. Drug Discov. 9 (2010): 560-574). The availability of such a biomarker would be of outmost importance for the development of disease-modifying therapies. The earlier such therapies would be administered, the bigger the chances for success. And, one could limit such efforts to true proteinopathies, such as PD and MSA, identified with the help of a specific biomarker.

It is an object of the present invention to provide means for improving the diagnosis of proteinopathies, including PD/LBD, DLB, PDD, PAF, RBD, MSA, NBIA type1, IBM, AD, DS, PSP, CBD and FTD/PiD, ALS and T2D, especially for tracking early stages of the disease and for observing the development of clinical trials for drugs for the treatment of proteinopathies. It is a further object to provide reliable tools for detecting proteinopathy-specific antibodies in biological samples, especially in samples of human synucleinopathy patients or human individuals who are suspected to have or are at risk of developing a proteinopathy, especially a synucleinopathy, including PD/LBD, DLB, PDD, PAF, RBD, MSA, NBIA type1 and IBM.

Therefore, the present invention provides a method for detecting proteinopathy-specific antibodies in a biological sample comprising the following steps:
- contacting the sample with aggregates, comprising two or more of a protein species selected from the group consisting of alpha synuclein (aSyn) or an aggregate-forming variant thereof, Tau protein or an aggregate-forming variant thereof, amyloid beta (Aβ)1-42 peptide or an aggregate-forming variant thereof; or contacting the sample with particles having aggregate like surfaces comprising two or more of said protein species, and allowing the proteinopathy-specific antibodies to bind to the aggregates, and
- detecting the proteinopathy-specific antibodies bound to the aggregates by a single particle detection technique, preferably by fluorescence activated cell sorting (FACS).

With the present invention, a new method to detect proteinopathy-specific antibodies, especially proteinopathy-specific auto-antibodies is disclosed which can be used as diagnostic tool which is extremely helpful in diagnosing of proteinopathies, especially synucleinopathies, including PD/LBD, DLB, PDD, PAF, RBD, MSA, PSP, CBD, FTD/PiD and NBIA type 1 and monitoring the development of these diseases, as well as for diseases like AD, DS, PSP, CBD and FTD/PiD, ALS and T2D. The present method is based on the invention that not just single proteinopathy peptides are used as capturing tools for the proteinopathy-specific antibodies, but that instead mixed aggregates of proteinopathy-protein species are used and that thereby generated antibody-aggregate complexes are detected using a single particle detection technique.

aSyn (initially identified as PARK1 and PARK4) is a 140 amino acid protein widely expressed in the neocortex, hippocampus, dentate gyrus, olfactory bulb, striatum, thalamus and cerebellum as well as in peripheral neurons and ganglia, e.g.: in colon or muscle tissue. The major form of the protein is the full 140 amino acids-long transcript. Other isoforms are alpha-synuclein-126, where exon 3 is lost and lacks residues 41-54; and alpha-synuclein-112, which lacks residue 103-130 due to loss of exon 5 and alpha-synuclein-98, lacking exon 3 and 5. aSyn is also highly expressed in hematopoietic cells including B-, T-, and NK cells as well as monocytes and platelets. The exact role in these cells is not known but it has been implicated in the differentiation of megakaryocytes (platelet precursors).

Beside the full length form of the protein, different forms of modified aSyn have been identified including phosphorylated, nitrosylated/nitrated, acetylated and mono-, di-, or tri-ubiquitinated aSyn. In addition, C-terminally truncated forms of the protein, like aSyn 1-119, aSyn 1-122 and aSyn 1-123, have been detected in brain tissue from both transgenic mice and PD/MSA cases.

It is currently believed that up to 15% of the aSyn detected in LBs and lewy neurites or GCIs is truncated. Previous in vitro studies using truncated aSyn could demonstrate that aSyn lacking the C-terminal 20-30 amino acids was showing an increased tendency to aggregate and to form filaments found in Lewy-neurites and LBs. In addition, approximately 90% of aSyn deposited in LBs is phosphorylated at serine 129 (Ser129). In contrast, only 4% of total aSyn is phosphorylated in normal brain, suggesting that accumulation of Ser129-phosphorylated aSyn might be crucial in the pathogenesis of PD. Therefore, these truncated and/or modified forms of aSyn are thought to act as seed molecules for formation aSyn oligomers/fibrils. Thus full length aSyn as well as truncated and/or modified forms of aSyn are then believed to accumulate leading to aggregate-formation. Based on recent studies it is believed that such aggregate-formation, (i.e. oligomers and fibrils) for example in the synaptic terminals and axons, plays an important role for PD/LBD/synucleinopathy development and could thus be enhanced by the presence of truncated/modified forms of aSyn.

Preferably the aggregates according to the present invention comprise aSyn or an aSyn variant. Full-length aSyn protein is the 140 aa long protein as disclosed above. aSyn-comprising-aggregates contain aSyn protein besides other components, especially other amyloidogenic polypeptides, such as Aβ1-42, Aβp(E)3-42 (or other Aβ forms), Tau protein (including phosphorylated Tau) . The aggregates according to the present invention may contain - instead or besides aSyn (the full length aSyn 140 aa protein) - aSyn variants, i.e. aSyn polypeptides that are truncated or modified, especially aSyn polypeptides that are naturally occurring forms of aSyn, such as aSyn-98 (lacking exon 3 and 5), aSyn-126 (lacking aa 41-54; loss of exon 4) and aSyn-112 (lacking aa 103-130; loss of exon 5), phosphorylated (especially at serin 129 - Ser129), nitrosylated/nitrated, acetylated and mono-, di-, or tri-ubiquitinated aSyn, or C-terminally truncated forms of aSyn such as aSyn lacking the C-terminal 20, preferably lacking the C-terminal 25, especially lacking the C-terminal 30 amino acids. The prerequisite for such aggregate components, especially the aSyn variants disclosed above, is that these components are capable of forming aggregates (i.e. capable of forming aggregates under conditions applied according to the disclosure according to the present invention, especially in the example section).

The "Aβ1-42-variants" (or "Aβ-variants", as synonymously used herein, since Aβ1-42 is the most preferred aggregate forming polypeptide of this group; accordingly, also Aβ-1-43 is defined as an "Aβ1-42-variant" herein used according to the present invention are selected from the group consisting of
- a mutant of the Aβ-1-40- or the Aβ-1-42-peptide containing at least one mutation at an amino acid position other than amino acids 16 to 20, 25 to 28 and 31 to 35,
- a truncated form of the Aβ-1-40- or the Aβ-1-42-peptide, wherein the truncation is at amino acid positions other than amino acids 16 to 35 and wherein the truncation is at least one amino acid and at most 20 amino acids; or
- combinations thereof, especially combinations of at least one mutation and at least one truncation of the Aβ-1-40-or the Aβ-1-42-peptide.

Preferred Aβ1-42-peptide variants according to the present invention include truncations and/or modifications of Aβ1-40/42 (i.e. peptides containing amino acid exchanges; the number of exchanges may be more than 10, however, preferred variants have 10 to 1 exchanges, more preferred 5 to 1 exchanges, even more preferred 4, 3, especially a single exchange.

The following groups of Aβ variants are specifically preferred in Aβ-variant aggregates (group 4 being the most preferred, followed by group 3, then group 2 and group 1)

Group 1 of Aβ variants: peptides containing either the core sequence Aβ16-20 or the second core sequence Aβ25-35. These peptides have been shown to form amyloid fibrils by themselves. Starting from these core sequences peptides can be extended by amino acids present in Aβ peptide or by alternative amino acids forming either truncated native Aβ peptides or truncated-modified Aβ peptides.

Group 2 of Aβ variants: peptides containing the sequence Aβ16-20-X₂₁₋₂₃-24-28-X₂₉₋₃₀-31-36 (meaning that aa 16 to 20, 24 to 28 and 31 to 36 are present and linked by a 3 and a 2 aa linker (of arbitrary primary sequence)). Starting from this sequence peptide can be extended by amino acids present in Aβ1-42 peptide or by alternative amino acids forming either truncated native Aβ peptides or truncated-modified Aβ peptides.

Group 3 of Aβ variants: peptides containing the sequence Aβ16-36. Starting from this sequence peptide can be extended by amino acids present in Aβ peptide or by alternative amino acids forming either truncated native Aβ peptides or truncated-modified Aβ peptides.

Group 4 of Aβ variants: peptides containing the sequence Aβ11-36 or Aβp(E)11-36. Starting from this sequence peptide can be extended by amino acids present in Aβ peptide or by alternative amino acids forming either truncated native Aβ peptides or truncated-modified Aβ peptides.

Mutations are preferably conservative mutations, i.e. amino acid mutations of residues having similar side chains. A conservative mutation substitutes an amino acid in the polypeptide with an amino acid within the same or similar defined class of amino acids. For example, A, V, L, or I can be conservatively mutated to either another aliphatic residue or to another non-polar residue. Conservative amino acid substitutions can frequently be made in a polypeptide without altering either the conformation or the function of the polypeptide. Substitutions can be chosen based on their potential effect on (a) backbone structure in the vicinity of the substitution, for example, a sheet or helical conformation, (b) the charge or hydrophobicity of the molecule at the target site, or (c) the volume and branching of the side chain. In general, amino acid residues can be classified based on side-chain properties: (1) aliphatic: A, M, V, L, I; (2) small aliphatic: A, V; (3) large aliphatic: M, L, I; (4) neutral hydrophilic: S, T, N, Q; (5) acidic: D, E; (6) basic: H, K, R; (7) charged: R, D, E, H, K; (8) residues that affect backbone conformation: G, P; and (9) aromatic: W, Y, F. Non-conservative mutations substitute a member of one of these classes for a member of a different class. Conservative mutations according to the present invention substitute a member of one of these classes for another member of the same class (except class (8)). Generally mutations are not made to C, G and P.

Preferred aa positions that are mutated according to the present invention are between 11 to 16 and 36 to C-terminus (e.g. 40, 42). Here, also non-conservative exchanges (preferably to small neutral or aliphatic amino acids) can be performed. In the loop region it is preferred to only provide (conservative) mutations at amino acid residues 21 to 23 and 29 to 30. In the N-terminal region (e.g. residues 1-10), virtually all mutations and truncations are possible (conservative and non-conservative), aggregate formation according to the present invention is usually not affected by this region.

Preferably, the aggregate-forming aSyn variant, the aggregate-forming Tau protein variant and the aggregate-forming Aβ1-42 variant is therefore, independently of each other, selected from the following groups:
(1) aSyn-126 (lacking aa 41-54; loss of exon 4), aSyn-112 (lacking aa 103-130; loss of exon 5), aSyn-98 (lacking exon 3 and 5), phosphorylated aSyn, aSyn-126, aSyn-98 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids,
(2) hyperphosphorylated Tau, abnormally phosphorylated Tau (as referred to and defined in Shahani et al. J. Neurosci. 26 (2006), 6103-6114), Tau protein variants that have marker function for a disease (either isoform of all 6 naturally occurring isoforms (Tau441, Tau412, Tau410, Tau383, Tau381, Tau352) or mutant forms thereof (e.g. P301L, P301S, V337M, etc.), e.g.: forms preferably simultaneously phosphorylated at amino acids 181, 202, 205, 212, 214, 231, 396 and, optionally at additional residues present in Tau like 18, 153, 175, 189, 235, 262, 394, 404 and 422, of Tau441, Tau412, Tau410, Tau383, Tau381, Tau352 (a more detailed analysis of Tau phosphorylation is disclosed e.g. in Hanger et al., Trends in Mol. Med. 15 (2009), 112-119); and
(3) Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42; an Aβ variant selected from the group consisting of
   - a mutant of the Aβ-1-40- or the Aβ-1-42-peptide containing at least one mutation at an amino acid position other than amino acids 16 to 20, 25 to 28 and 31 to 35,
   - a truncated form of the Aβ-1-40- or the Aβ-1-42-peptide, wherein the truncation is at amino acid positions other than amino acids 16 to 35 and wherein the truncation is at least one amino acid and at most 20 amino acids; or
   - combinations thereof, especially combinations of at least one mutation and at least one truncation of the Aβ-1-40- or the Aβ-1-42-peptide.

Preferred embodiments of the present invention are mixed aggregates comprising at least two species of aSyn and an aggregate-forming variant thereof, or Tau protein and an aggregate-forming variant thereof, or Aβ1-42 peptide and an aggregate-forming variant thereof. Moreover, other aggregate binding polypeptides may be present in the aggregates according to the present invention, preferably IAPP and an aggregate-forming variant thereof, especially IAPP-Npro, TAR-DNA binding protein 43 (TDP43) or an aggregate-forming variant thereof; Superoxide-Dismutase 1 (SOD1) or an aggregate-forming variant thereof, specifically allowing also the detection of IAPP-, TDP43- and SOD1-specific antibodies in patients with the present invention

IAPP is synthesized as a 89 amino acid residue pre-prohormone. Cleavage of a signal sequence leads to a pro-IAPP containing 67 amino acids. The latter is further processed to the 37 residue mature IAPP. It has been shown that normal processing of pro-IAPP is a two-step process initiated by cleavage at its COOH terminus (likely by prohormone convertase 1/3 in the trans-Golgi network) followed by cleavage at its NH2 terminus (by prohormone convertase 2 in granules) to form the mature form of IAPP. IAPP is strongly conserved among mammalian species but exhibit notable variation between the amino acids 20 and 29. The differences in this region determine the ability of IAPP to aggregate and to form amyloids. In fact, species with one or more proline residues (except cats) at this region do not form islet amyloids. IAPP was discovered through its ability to aggregate into pancreatic islet amyloid deposits, which have been associated with β-cell dysfunction and death in Diabetes mellitus Type 2 (type 2 diabetes; T2D) patients and in other mammalian species like monkeys and cats. Amyloids are insoluble, fibrillar, protein aggregates with a β-pleated sheet structure. Amyloids have characteristic optical properties after staining with Congo red or thioflavin S and can be analyzed by light microscopy. However, the development of amyloid starts with the formation of fibrils or aggregates (7-10 nm in diameter and hundreds of nm in length) which can be detected by electron microscopy before evidence of amyloid is observed by light microscopy. In T2D patients, histological analyses of pancreatic tissue indicate that an accumulation of IAPP aggregates leads to the replacement of β-cell mass, resulting in progressive β-cell dysfunction and hyperglycemia. Besides that, IAPP fibrils appear to have a direct damaging effect on islets. Pancreatic amyloid deposits are found in more than 95% of type II diabetes patients and their formation is strongly associated with disease progression. In general, amyloids can form from proteins that fold to a compact tertiary structure in their unaggregated state, or they can originate from intrinsically disordered polypeptides that fail to adopt compact tertiary structures in their soluble native state. IAPP is an important example of intrinsically disordered polypeptide that forms amyloid in vivo. Although IAPP is major component of the islet amyloids in the pancreas of T2D patients, at least two additional elements have been identified in these deposits: apoE and the heparin sulfate proteoglycan perlecan (HSPGs). The kinetics of amyloid formation is complex and displays a sigmoidal profile characterized by a final steady state where soluble peptide is at equilibrium with amyloid fibrils. Amyloid formation can be accelerated by the addition of small amounts of preformed fibrils in a process called "seeding". A fraction of the IAPP that is secreted in T2D is incompletely processed. The impaired NH₂-terminal processing of pro-IAPP leads to amyloid formation and β-cell death. Accumulation of the NH₂-terminally extended human pro-IAPP intermediate (IAPP-Npro) may be a critical initiating step in amyloid formation. In fact, although IAPP-Npro was found to be less amyloidogenic in solution than mature IAPP, it interacts more effectively with glycosaminoglycans (GAGs), a component of HSPGs. Accordingly, IAPP forms with impaired NH₂-terminal processing, especially IAPP-Npro, are preferred forms in aSyn-comprising-aggregates according to the present invention.

Accordingly, aggregates according to the present invention are e.g. a mixed aggregate of Aβ1-42 and Aβ1-40, a mixed aggregate of Aβ1-42 and Aβ1-43, a mixed aggregate of Aβ1-42, Aβp(E)3-42 and Aβ1-40, a mixed aggregate of phosphorylated aSyn and aSyn-126, a mixed aggregate of aSyn, phosphorylated aSyn and aSyn-126, a mixed aggregate of hyperphosphorylated Tau and Tau, a mixed aggregate of hyperphosphorylated Tau and Tau410, a mixed aggregate of hyperphosphorylated Tau, Tau and asyn, or a mixed aggregate of hyperphosphorylated Tau and one or more of IAPP, TDP43 and SOD1. Also such aggregates can, of course, contain protein species from other groups (which resembles the situation in patients usually more accurately, since also in patients, the protein deposits may consist of a mixture of different polypeptide species. However, for certain embodiments it can be preferred to use only polypeptide species from one group in the mixed aggregates.

According to a preferred embodiment, the proteinopathy-specific antibodies are selected from the group consisting of anti-aSyn antibodies, anti-Tau antibodies and anti-Aβ antibodies.

The mixed aggregates according to the present invention are generated e.g. by overnight incubation of the aggregate-forming proteins, e.g. aSyn or an aggregate-forming variant thereof, Tau protein or an aggregate-forming variant thereof, amyloid beta Aβ1-42 peptide or an aggregate-forming variant thereof; provided that at least two different aggregate-forming polypeptide species are aggregated. It is possible to combine various polypeptide species in the mixed aggregates according to the present invention, preferably to resemble the situation in pathophysiological circumstances in patients. Proteinopathies are characterised by aggregation of certain polypeptide species; the mixed aggregates according to the present invention can provide a realistic model for such in vivo aggregates and are therefore suitable to detect, identify and isolate the disease-specific antibodies present in patients that have been suspected to have or already diagnosed to have a proteinopathy. Such diseases may even be determined in a very early stage, because disease-characteristic antibodies or disease-characteristic antibody concentration e.g. in blood may be diagnosed at a significant term before outbreak of the disease of before symptoms of the disease are detectable.

After generation of the mixed aggregates according to the present invention e.g. by overnight incubation of the aggregate-forming polypeptides (as used herein, the term "polypeptides" is covering the term "protein" and usually used synonymously; the term "protein" is therefore applied herein also for polypeptides with less than 100 or less than 50 amino acid residues), the mixed aggregates are subsequently incubated with serum samples derived either from healthy donors (HD) or from proteinopathy patients, to allow binding of present antibodies (both IgG and IgM). Antibodies bound to the aggregates can be detected by any suitable method available to a person skilled in the art, e.g. by using a labelled secondary antibody which recognises the proteinopathy-specific antibody bound to the aggregates. For example a phycoerythrin (PE)-labelled secondary antibody can be used. Thereafter, the immune complexes comprising the proteinopathy-specific antibody bound to the aggregates (and optionally one or more detection agents, such as secondary antibodies) are measured using a single particle detection technique, such as FACS (fluorescence activated cell sorting)-analysis also known as flow cytometry. The level of proteinopathy-specific antibodies obtained for a given sample can then be compared to the level of a healthy sample or to the level in a sample of a patient with a known disease status, for example the level in a sample of a PD or AD patient. Using the method according to the present invention, it can be shown that proteinopathy patients contain different levels of proteinopathy-specific immunoglobulins which are reactive towards the aggregates provided according to the present invention compared to healthy subjects. Furthermore, using the method according to the present invention, it can be shown that reactivity of proteinopathy-specific immunoglobulins (towards the aggregates provided according to the present invention) derived from proteinopathy patients can be increased by a procedure known as demasking (removing of potentially bound proteinopathy-antigens from autoantibodies). This is in contrast to the reactivity of proteinopathy-specific immunoglobulins from healthy subjects where such an increase of reactivity towards the aggregates cannot be detected after treating these sera in a special way. On the other hand, the reactivity of IgM-antibodies after demasking (=e.g.: dissociation of already bound aSyn in the serum) revealed an increased level of IgM in proteinopathy patients. Additionally, also the difference between IgG levels with and without demasking was determined (delta (Δ) values). This parameter was also elevated in proteinopathy patients as compared to healthy controls showing higher antibody occupancy in the pathological state of the disease. Furthermore, with the present invention data are provided showing that the present method has a much higher capacity to detect proteinopathy-specific antibodies and thus has a much higher power to diagnose proteinopathies, such as PD or AD, compared to methods published so far. Given these facts, the method according to the present invention fulfils the theoretical prerequisites of a predictive diagnostic tool to identify proteinopathies, preferably synucleinopathies, especially PD, and to follow the clinical response of a given patient to treatment.

The present invention was developed for the analysis of proteinopathy-specific antibodies in human samples. It is therefore a preferred embodiment to detect human proteinopathy-specific antibodies, preferably human IgG or IgM antibodies, especially human IgG antibodies. As already mentioned, the detection of proteinopathy-specific antibodies in human is known in principle in the art; however, the role as a possible biomarker could not be verified. As shown with the present invention, this was also due to the analytical insufficiency of the detection methods available in the art. Due to the superiority of the method according to the present invention, the availability of these antibodies in human samples as biomarkers is enabled. The present method is therefore specifically suited to detect autoantibodies in biological samples. Accordingly, in a preferred embodiment of the present method, the proteinopathy-specific antibodies to be detected and quantified are autoantibodies.

In contrast to prior art methods, the present method uses mixed aggregates as probe for binding the proteinopathy-specific antibodies from the samples. Apart from the fact that such aggregates have not yet been provided in the prior art, it was also not realised that the use of such aggregates in the analysis of proteinopathy-specific antibodies, especially in human samples, could significantly improve such methods, also in combination with the single particles detection techniques, such as FACS. Due to the use of such aggregates, the detection with single particles detection techniques (which are established techniques in various different fields and for different questions) is possible for analysing proteinopathy-specific antibodies in human samples (such as blood) which are usually very complex and difficult to handle.

Preferably, the dimensions of the aggregates to be used according to the present invention are standardised for analytical use. This can be done by establishing certain parameters during production of the aggregates. Depending on the conditions applied during generation of the aggregates, the size of the aggregates can be adjusted. Preferred sizes of the aggregates according to the present invention are from 50 nm to 15 µm, preferably from 100 nm to 10 µm, especially from 500 nm to 5 µm (defined by the length of the aggregates (i.e. the longest extension).

According to the present method, the samples wherein the proteinopathy-specific antibodies are to be detected are contacted with the mixed aggregates according to the present invention to achieve binding of the proteinopathy-specific antibodies possibly present (and reactive vis-a-vis aggregates) in the samples. The concentration of the aggregates has therefore to be adjusted in order to provide enough binding positions for the antibodies. Accordingly, the concentration of the aggregates for binding the antibodies in the sample is preferably in the range of 0.001 to 100 µM, preferably 0.01 to 10 µM, especially 0.1 to 1 µM. The optimal concentration is also dependent on the nature of antibodies to be bound, the nature of the sample, the planned contact time and the size of the aggregates.

The present method is mainly aimed for application on human samples. It is therefore preferred that biological sample is human blood or a sample derived from human blood, preferably human serum or human plasma; human cerebrospinal fluid or human lymph. With such sample sources, also serial and routine testing may be established (especially for samples derived from blood).

Preferred contact times which allow proper binding of the antibodies in the sample to the aggregates are at least 10 min (e.g. 10 min to 48 h), preferably from 15 min to 24 h, especially from 30 min to 2 h.

If the biological sample is not specifically pre-treated, the proteinopathy-specific antibodies which have a binding capacity to the aggregates will be bound during contact with the aggregates. The proteinopathy-specific antibodies which are masked in the sample (i.e. those antibodies which are already bound to a binding partner) will not be detected by the method according to the present invention (absent such specific sample pre-treatment). Whereas the identification and quantification of only reactive antibodies will in many cases be sufficient and desired, there may be situations or diagnostical questions where the total amount of proteinopathy-specific antibodies in the sample should be detected (reactive and unreactive) or all, the number of reactive proteinopathy-specific antibodies, the unreactive ("masked") and the total number of proteinopathy-specific antibodies.

Therefore, according to another preferred embodiment of the present invention, the samples are demasked, i.e. the proteinopathy-specific antibodies are "freed" from any binding to binding partners present in the sample previous to the contact with the aggregates according to the present invention. This allows detection of all proteinopathy-specific antibodies in the sample and not only detection of those antibodies which are not bound to a binding partner in the sample ("free" or "reactive" antibodies). In the course of the present invention it was determined that the amount of reactive proteinopathy-specific antibodies, especially reactive proteinopathy-specific IgG, was a key marker for the diagnosis and development of the proteinopathy, especially PD and MSA. The present method is, as stated above, also suitable for determining the overall amount of proteinopathy-specific antibodies in a sample, i.e. the free (or "reactive") antibodies as well as those antibodies which are already bound in the sample. This can be helpful in establishing the difference (Δ) of reactive vs. non-reactive antibodies in a sample, a parameter which is also of significant importance for proteinopathy, especially PD and MSA, diagnosis. Whereas such difference is not present (or low) in persons with "healthy status" concerning proteinopathy, especially PD and MSA, this difference is crucial for the marker function in e.g. PD and MSA, concerning both proteinopathy-specific IgG and proteinopathy-specific IgM.

The method according to the present invention applies a single particle detection technique. Such techniques allow identifying and quantifying ("count") the number and amount of "positive" binding results of the proteinopathy-specific antibody to the mixes aggregates according to the present invention. A preferred embodiment of this technology is FACS which is an established technique in the present field. Other detection methods to be used to detect the antibodies bound to the present aggregates are e.g. Luminex or mass cytometry.

According to the Luminex technology, sample preparation may be performed as described in Material and Methods. Following sample preparation the mixed aggregates according to the present invention recognized by specific proteinopathy-specific antibodies may be detected by a secondary antibody coupled to fluorescent-dyed microspheres which can be detected in multiplex detecting systems e.g. a Luminex reader (Binder et al., Lupus15 (2005):412-421).

If mass cytometry is used as single particle detection technique, sample preparation may also be performed as described in Material and Methods of the example section below. Sample preparation is done as described. Following sample preparation aggregates recognized by specific Abs may be detected by a secondary antibody coupled to stable isotopes of transition elements which can be detection by atomic mass spectrometry. The sample can then be sprayed through an argon plasma filled inductive coil heated to a temperature of >5,500 K. The sample is vaporized and ionized into its atomic constituents, and the number of the isotope-tagged antibody is quantified by time-of-flight mass spectrometry (Janes et al., Nat. Biotechnol. 29 (2011): 602-604).

Alternatively it is also possible to apply single particle detection techniques where one binding partner (aggregates or antibody/serum) are immobilized but binding is measured under flow conditions. Examples are the Hybcell-technology and the Surface Plasmon Resonance technology. Using the Hybcell technology in the present invention, serum samples can be spotted on the surface of the Hybcell (a rotating cylinder) and incubation can be performed with directly fluorescence-labelled preincubated aggregates or alternatively with a fluorescence-labelled monoclonal proteinopathy-specific second antibody. Antibodies bound to aggregates are detected with a laser (Ronacher, Anagnostics Technical Note ANA-TN-005 (2010)). If Surface Plasmon Resonance is used in the method according to the present invention, a reverse setup can be applied: the preincubated aggregates can be immobilized on a chip surface. The binding of proteinopathy-specific antibodies from serum to the aggregates on the chip can be detected by increase of mass on the chip surface and therefore no labelling of the binding partners is necessary. To increase sensitivity or determine IgG-subtypes a serial injection of anti-IgG-AB is possible (Cannon et al., Anal. Biochem. 328 (2004): 67-75). Instead of directly immobilizing aggregates to the chip surface a capture antibody can be used. For this setup a proteinopathy-specific antibody is immobilized on the chip surface followed by the injection of preincubated aggregates. After the capturing of the aggregates serum is injected and reactivity is measured by increase of mass.

Detection of the binding of proteinopathy-specific antibodies to the aggregates according to the present invention can be performed by any suitable method known e.g. Fluorescence Spectroscopy (Missailidis et al., Methods in Molecular Biology 248 (2003): 431-441) for detecting the proteinopathy-specific antibodies bound to the aggregates by a secondary antibody (e.g. a secondary labelled anti-IgG- or anti-IgM-antibody).

Detection of autoantibodies bound to aggregates can also be performed using substrates specifically binding antibodies such as Protein A or Protein G. Another possibility is to precipitate aggregate specific autoantibodies using the aggregates, wash the complex and biotinylate the antibodies. Subsequently streptavidin can then be used as second step reagent.

According to a preferred embodiment of the present invention, the particles having aggregate like surfaces are beads with aggregates immobilised on their surface, especially magnetic beads.

A preferred field of use of the present invention is the diagnosis of Synucleinopathies, including Parkinson's disease (PD), Lewy Body Disease (LBD), Dementia with Lewy Bodies (DLB), Parkinson's Disease Dementia (PDD), Multiple System Atrophy (MSA), Pure Autonomic Failure (PAF), REM Sleep Behaviour Disorder (RBD), Neurodegeneration with Brain Iron Accumulation type I (NBIA Type I) and inclusion body myositis (IBM); as well as other diseases, such as Alzheimer's disease (AD) and Down Syndrome (DS), Progressive Supranuclear Palsy (PSP), Cortico-basal degeneration (CBD), Frontotemporal Dementia /Pick's Disease (FTD/PiD).

A preferred embodiment of the present invention is the diagnosis of proteinopathies that is significantly improved by the present invention. Proteinopathies are, as already stated, diseases, especially neurodegenerative diseases that are caused by or connected to a malformed protein. Preferred proteinopathies to be diagnosed according to the present invention are Alzheimer's disease (AD), Dementia in Down's Syndrome, Parkinson's disease (PD), Lewy Body Disease (LBD), Dementia with Lewy Bodies (DLB), Parkinson's Disease Dementia (PDD), Multiple System Atrophy (MSA), Pure Autonomic Failure (PAF), REM Sleep Behaviour Disorder (RBD), Neurodegeneration with Brain Iron Accumulation type I (NBIA Type I), inclusion body myositis (IBM), Cortico-Basal Degeneration (CBD), Progressive Supranuclear Palsy (PSP), Pick's Disease (PiD), Dementia pugilistica (chronic traumatic encephalopathy, DP), Frontotemporal dementia (FTD), Lytico-Bodig disease (LD), Huntington's disease (HD) and Spinocerebellar ataxias (Type 1, 2,3 and 7) and Amyotrophic lateral sclerosis (ALS), prionosis and type II diabetes; and diseases with alpha synuclein deposition and/or aggregation, especially Alzheimer's disease (AD) Down Syndrome (DS), Progressive Supranuclear Palsy (PSP), Corticobasal degeneration (CBD), Frontotemporal Dementia/Pick's Disease (FTD/PiD), ALS and T2D; especially for tracking early stages of the proteinopathy and for observing the development of clinical trials for drugs for the treatment of proteinopathies.

The present method is specifically suited for using in connection with AD, PD and MSA diagnosis. With the present invention, proteinopathy-specific autoantibodies in human patients are provided as markers for AD, PD and MSA status. People with "normal" level of proteinopathy-specific antibodies in their blood are "healthy" with respect to PD and MSA. If this level is modified in a patient with PD and MSA or subjects with a risk of developing PD and MSA or being suspected to have AD, PD and MSA, such modification level correlates with AD, PD and MSA. A "modified" level may be a modification of the absolute number of the proteinopathy-specific antibodies or a modification of the reactivity of the totality of the proteinopathy-specific antibodies (e.g. of a given class of proteinopathy-specific antibodies (IgG, IgM, etc.). For example, modified reactive proteinopathy-specific IgG correlates with and is a marker for PD and MSA. With the present method, when the "healthy" level of reactive proteinopathy-specific IgG is set to 100%, a significant modification in reactive proteinopathy-specific IgG, is e.g. a decrease to 70% and lower, to 50% and lower or to 30% and lower, or an increase of at least 30%, e.g. at least 50% or at least 100%, in a blood sample.

Since the present invention provides a marker for PD and MSA and even for the development of AD, PD and MSA, it is possible to use this method for observing the development of the disease and the performance of possible treatment methods, especially whether the method of treatment enables to establish "healthy" or "healthier" levels of proteinopathy-specific antibodies, especially IgG or IgM.

The present method is therefore preferably used for the monitoring of AD, PD and MSA patients, especially PD and MSA patients who are treated with medicaments for curing or ameliorating AD, PD and MSA. The present method can be successfully applied for observing patients in clinical trials for AD, PD and MSA vaccines (e.g. PD01A, Trial AFF008, NCT 01568099) or aSyn-targeting disease-modifying drugs.

The method according to the present invention can also be used for evaluating the risk of developing a proteinopathy (especially AD), preferably a synucleinopathy, especially PD, or for detecting early stage of a proteinopathy (especially AD), preferably a synucleinopathy, especially PD and MSA. With the present invention, it is in principle made possible to detect changes in the immunological set-up of patients with respect to proteinopathy-specific autoantibodies at a significantly earlier point in time than cognitive/functional impairments. This could allow a significant improvement of early diagnosis of a proteinopathy (especially AD), preferably a synucleinopathy, especially PD and MSA, with respect to a much larger part of the population if established in routine testing format. This makes patients eligible for early stage treatment regimens and/or prevention (or delay) strategies for proteinopathies (especially AD), preferably synucleinopathies, especially PD and MSA, especially immunotherapy (including vaccination).

According to another aspect, the present invention relates to a kit for performing the method according to the present invention comprising
- mixed aggregates according to the present invention, and
- a sample container, especially for human samples (e.g. blood, serum, plasma).

Preferably, the kit according to the present invention may further contain means for detecting the mixed aggregates being bound to proteinopathy-specific antibodies, preferably secondary antibodies, especially labelled secondary antibodies, e.g. anti-IgG- or anti-IgM-antibodies). Further components can be standard samples, positive and/or negative controls, instructions for use and suitable packaging means (e.g. stable boxes, coloured vials, etc.).

According to another aspect, the present invention relates to an aggregate, comprising two or more of a protein species selected from the group consisting of alpha synuclein (aSyn) or an aggregate-forming variant thereof, Tau protein or an aggregate-forming variant thereof, and Aβ1-42 peptide or an aggregate-forming variant thereof.

The "aggregate" according to the present invention refers to the aggregate as such, but also to preparations containing the aggregate, especially preparations suitable for diagnostic purposes in human medicine, i.e. preparations made according to GMP and standardised procedures.

Preferably, the aggregate-forming aSyn variant, the aggregate-forming Tau protein variant, and the aggregate-forming Aβ1-42 variant in the aggregates according to the present invention are, independently of each other, selected from the following groups:
(1) aSyn-126 (lacking aa 41-54; loss of exon 4), aSyn-112 (lacking aa 103-130; loss of exon 5), phosphorylated aSyn, aSyn-126 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids,
(2) hyperphosphorylated Tau, abnormally phosphorylated Tau (as referred to and defined in Shahani et al. J. Neurosci. 26 (2006), 6103-6114), Tau protein variants that have marker function for a disease (either isoform of all 6 naturally occurring isoforms (Tau441, Tau412, Tau411, Tau383,Tau381, Tau352) or mutant forms thereof (e.g. P301L, P301S, V337M, etc.), e.g.: forms preferably simultaneously phosphorylated at amino acids 181, 202, 205, 212, 214, 231, 396 and, optionally at additional residues present in Tau like 18, 153, 175, 189, 235, 262, 394, 404 and 422, of Tau441, Tau412, Tau410, Tau383, Tau381, Tau352 (a more detailed analysis of Tau phosphorylation is disclosed e.g. in Hanger et al., Trends in Mol. Med. 15 (2009), 112-119); and
(3) Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42; an Aβ variant selected from the group consisting of
   - a mutant of the Aβ-1-40- or the Aβ-1-42-peptide containing at least one mutation at an amino acid position other than amino acids 16 to 20, 25 to 28 and 31 to 35,
   - a truncated form of the Aβ-1-40- or the Aβ-1-42-peptide, wherein the truncation is at amino acid positions other than amino acids 16 to 35 and wherein the truncation is at least one amino acid and at most 20 amino acids; or
   - combinations thereof, especially combinations of at least one mutation and at least one truncation of the Aβ-1-40- or the Aβ-1-42-peptide.

Preferably, the aggregate has a size of 50 nm to 15 µm, preferably from 100 nm to 10 µm, especially from 200 nm to 5 µm.

Preferably, the aggregate is obtainable by incubating the protein species at a pH of 2 to 9 for at least 20 min, preferably at least 1 h, especially at least 4 h.

According to a preferred embodiment of the present invention, the aggregate comprises three or more, preferably four or more, especially five or more, of a protein species selected from the group consisting of alpha synuclein (aSyn) or an aggregate-forming variant thereof, Tau protein or an aggregate-forming variant thereof, Aβ1-42 peptide or an aggregate-forming variant thereof, islet amyloid polypeptide (IAPP) or an aggregate-forming variant thereof, TAR-DNA binding protein 43 (TDP43) or an aggregate-forming variant thereof; Superoxide-Dismutase 1 (SOD1) or an aggregate-forming variant thereof.

Preferably, the aggregate comprises at least two protein species from different groups of the following groups (a) to (d):
(a) alpha synuclein (aSyn) or an aggregate-forming variant thereof,
(b) Tau protein or an aggregate-forming variant thereof,
(c) Aβ1-42 peptide or an aggregate-forming variant thereof,
(d) islet amyloid polypeptide (IAPP) or an aggregate-forming variant thereof, TAR-DNA binding protein 43 (TDP43) or an aggregate-forming variant thereof; Superoxide-Dismutase 1 (SOD1) or an aggregate-forming variant thereof.

According to a preferred embodiment, the aggregate comprises
- at least one protein species from group (a): alpha synuclein (aSyn) or an aggregate-forming variant thereof; and at least one protein species from group (b): Tau protein or an aggregate-forming variant thereof, or
- at least one protein species from group (a): alpha synuclein (aSyn) or an aggregate-forming variant thereof; and at least one protein species from group (c) Aβ1-42 peptide or an aggregate-forming variant thereof, or
- at least one protein species from group (b): Tau protein or an aggregate-forming variant thereof; and at least one protein species from group (c) Aβ1-42 peptide or an aggregate-forming variant thereof.

Preferably, the aggregate comprises aSyn and Aβ1-42 peptide, aSyn and hyperphosphorylated Tau protein, hyperphosphorylated Tau protein and Aβ1-42 peptide, or aSyn and Aβ1-42 peptide and hyperphosphorylated Tau protein.

The invention is further disclosed by the following examples and the figures, yet without being restricted thereto.

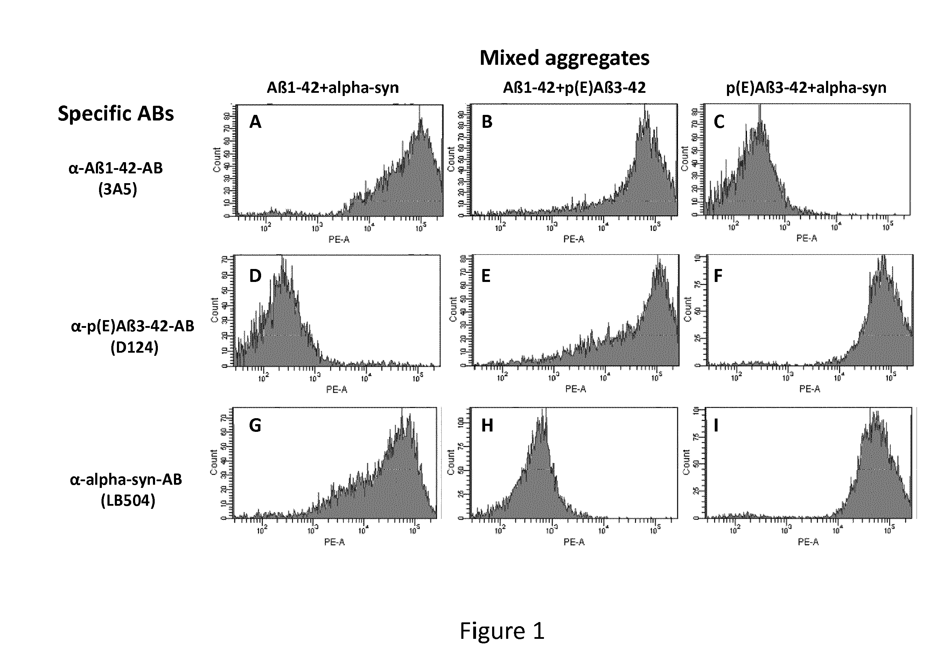

Figure 1 shows the reactivity of mAB against aggregates generated from equimolar mixtures of either aSyn with Aβ1-42, aSyn with p(E)Aβ3-42 or Aβ1-42 with p(E)Aβ3-42. Aggregates were incubated with monoclonal antibodies specific either for Aβ1-42 (3A5) p(E)Aβ3-42 (D129) and aSyn (LB509) and the mAB-binding pattern of the mixed aggregates was determined.

FACS PE-histograms show that the monoclonal antibody 3A5 binds exclusively mixtures which contain Aβ1-42 aggregates (1A+B) but no reactivity to the p(E)Aβ3-42/aSyn aggregate mixture (1C). The anti-p(E)Aβ3-42mAB D129 binds only mixtures that contain p(E)Aβ3-42 (1E+F) but shows no reactivity to Aβ1-42/aSyn (1D). The aSyn specific mAB LB509 reacts only with aggregate mixtures containing aSyn (1G+I) but does not react with the Aβ1-42/p(E)Aβ3-42 aggregate mixture (1H).

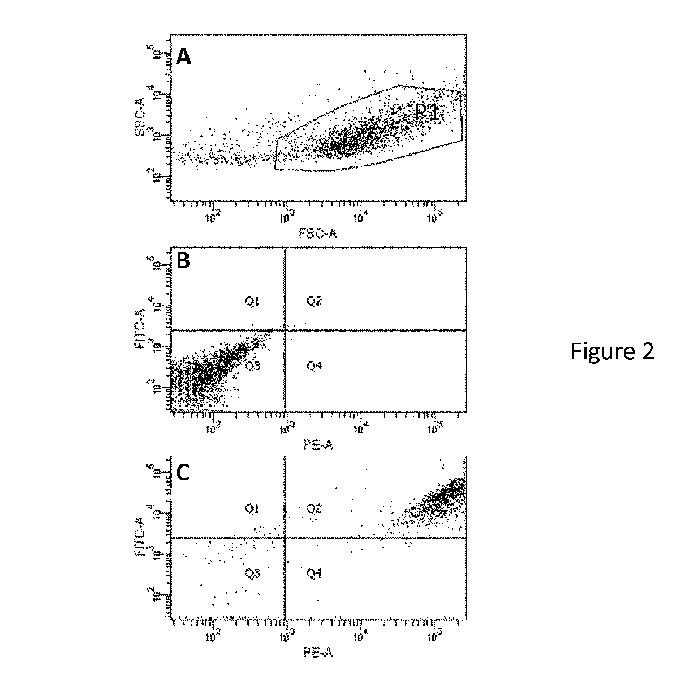

Figure 2: FACS analysis of aggregates generated with equimolar mixtures of differentially fluorescent-labelled Aβ1-42 and aSyn peptides of Aβ1-42-Hilyte-555 (PE) and aSyn-Hilyte-488 (FITC). Acquired aggregates with a size range of 0,5-10 µm were gated in P1 in the FSC-A/SSC-A (A) and P1 was further evaluated in the PE/FITC-dot plot to determine the contribution of Aβ1-42-Hilyte-555 (PE) and aSyn-Hilyte-488 (FITC) (C). Unlabelled mixed-aggregates were used as negative control (B).

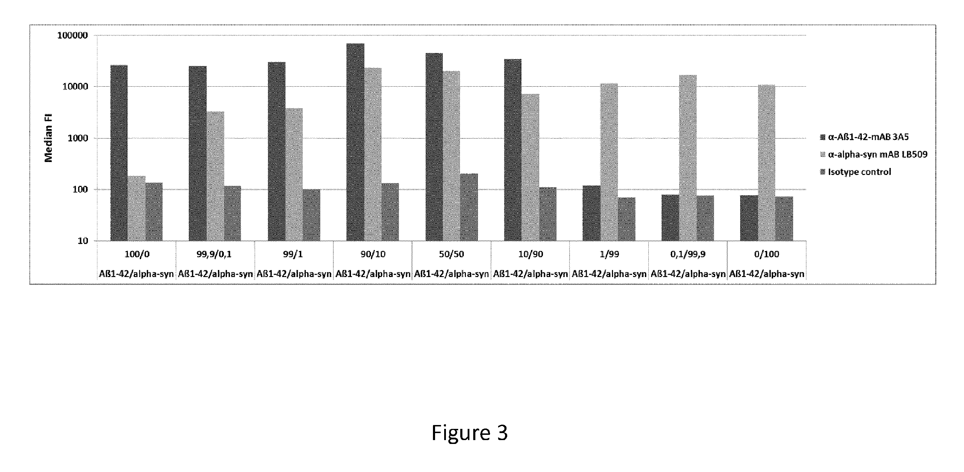

Figure 3: mAB reactivity against aggregates generated from different concentrations of aSyn and Aβ1-42. Aβ1-42 and aSyn peptides were mixed as indicated in table 1.

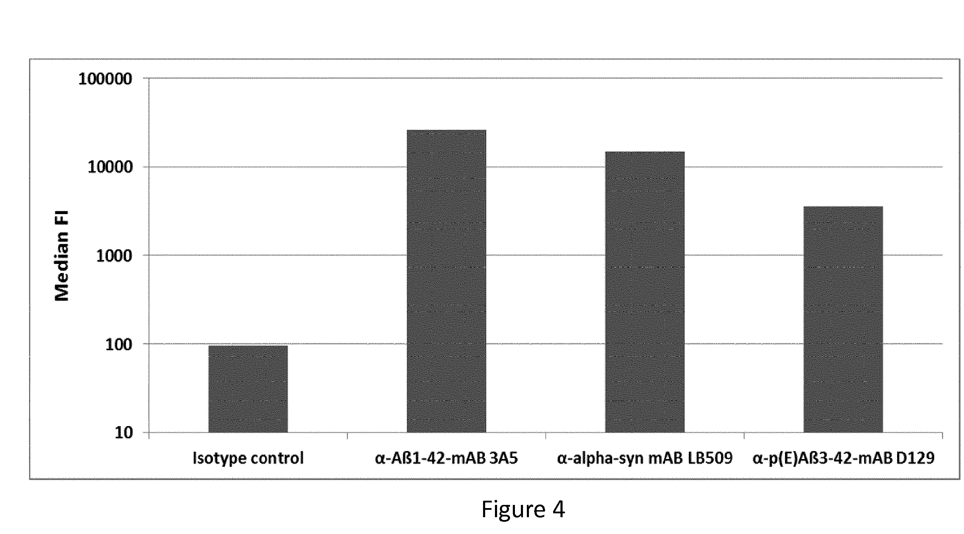

Figure 4 shows the reactivity of mAbs specific for Aβ1-42 (3A5), Aβp(E)3-42 (D129) and aSyn (LB509) against aggregates composed of an equimolar mixture of Aβ1-42, Aβp(E)3-42 and aSyn peptides.

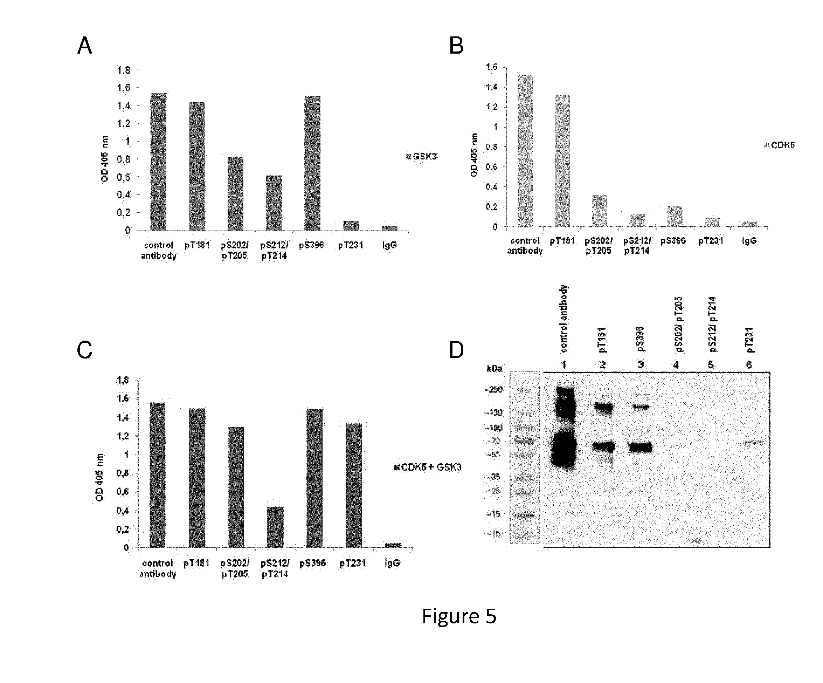

Figure 5 shows the analysis of phosphorylated recombinant human Tau - 441 protein with phosphorylation-dependent anti-Tau antibodies. Determination of different phosphorylation sites was done with an ELISA assay. Phosphorylation-dependent anti-tau antibodies are recognising phosphorylated serine or threonine residues at the corresponding sites. Control antibody (HT7) recognises the phosphorylated and unphosphorylated tau, IgG antibody was used as a control antibody for the ELISA assay. AT270 (pT181), AT8 (pS202, pT205), AT100 (pS212, pT214), PHF13 (pS396) and PHF6 (pS231) antibodies are showing a crossreactivity to their corresponding phosphorylation sites indicating the presence of multiple phosphorylation sites.

Different phosphorylation - dependent antibodies were used to determine the phosphorylation sites of tau after the incubation of tau with two different kinases, CDK5 and GSK3. Not only the phosphorylated sites of tau but also different aggregated tau forms were recognised, tau monomer, dimer and tetramer forms. Lane 1: a control antibody (HT7) recognises phosphorylated and unphosphorylated tau, Lane 2 - 6 were detected with phosphorylation - dependent antibodies.

### Example 1: Mixed aggregates:

### Materials and Methods

### Generation and detection of mixed aggregates

To test whether different peptides (e.g. aSyn and Aβ1-42) form mixed aggregates containing both peptides fluorescent N-terminally-labelled Aβ1-42 and C-terminally labelled aSyn peptides were used. As described by Anderson and Webb (BMC Biotechnology 2011, 11:125) labelling of Aβ and aSyn peptides does not prevent amyloid aggregate formation. Therefore, Aβ1-42-Hilyte-555 (detectable in the PE-channel) and aSyn-Hilyte-488 (detectable in the FITC-channel) were mixed at equimolar ratio (20µM) and incubated for 20h at room temperature on shaker (350 rpm) in 1% NH₄OH (pH 4,5). After incubation these aggregates were analysed using the FACS Canto II. Acquired aggregates were gated for size in P1 (∼0.5 µm - 10 µm) in the FSC-A/SSC-A and P1 was further analysed in the PE/FITC-dot plot to determine the distribution of Aβ1-42-Hilyte-555 (PE) and aSyn-Hilyte-488 (FITC).

### Detection of specific antibody reactivity by FACS analysis using aggregates generated from mixed solutions of monomeric aSyn, Aβ1-42 and p(E)Aβ3-42 proteins as a substrate

500 µg of lyophilized aSyn or 100 µg of either Aβ1-42 or p(E)Aβ3-42 was dissolved in 100µl of sterile filtered MonoQ (aSyn) or 100µl of sterile filtered 1% NH₄OH pH11 (Aβ1-42 and p(E)Aβ3-42) by resuspending and short vortexing until solution was clear. Subsequently these solutions were sonicated for 30 sec in a sonication water bath and stored in 5µl aliquots at - 20°C without shock-freezing. To induce the formation of aggregates, aliquots from individual proteins were thawed and the entire volume of the three individual protein solutions were diluted to an equimolar concentration of ∼35 µM in 1% NH₄OH (pH4,5). These protein solutions were mixed in all three possible combinations before incubation at 37°C on shaker (350 rpm) for 20h in an Eppendorf tube as follows: Aβ1-42 & aSyn, Aβ1-42 & p(E)Aβ3-42 and p(E)Aβ3-42 & aSyn. Furthermore, not only equimolar ratios (=50:50) but also a titration experiment to test different ratios of aggregate mixtures was performed. The ratios of aggregate mixes are indicated in the following

table 1 (e.g. 1:100=1 µl aSyn (20 µM) + 99 µl of Aβ1-42 (20 µM) solution) and the PE-MFI was determined after sample preparation.

**Table 1**

| | | % | µl |
|---|---|---|---|
| Substrate 1: | Aβ1-42/aSyn | 100/0 | 100/0 |
| Substrate 2: | Aβ1-42/aSyn | 99.9/0.1 | 99.9/0.1 |
| Substrate 3: | Aβ1-42/aSyn | 99/1 | 99/1 |
| Substrate 4: | Aβ1-42/aSyn | 90/10 | 90/10 |
| Substrate 5: | Aβ1-42/aSyn | 50/50 | 50/50 |
| Substrate 6: | Aβ1-42/aSyn | 10/90 | 10/90 |
| Substrate 7: | Aβ1-42/aSyn | 1/99 | 1/99 |
| Substrate 8: | Aβ1-42/aSyn | 0.1/99.9 | 0.1/99.9 |
| Substrate 9: | Aβ1-42/aSyn | 0/100 | 0/100 |

Mixed aggregates are used as substrates for further sample preparation and are treated as described for aSyn aggregates in paragraph above.

### Demasking

To disrupt the binding of aSyn specific auto-antibodies to aSyn likely present in patient sera and, therefore, preventing detection of these aSyn bound auto-antibodies by antigen-based methods (like ELISA or FACS), sera are pre-diluted in 10mM Glycin pH2.6 at a dilution of 1:16.7 for 5min. 5µl of the acidified serum are then co-incubated with 3µl of aSyn for another 5min. Then the mixture is neutralized by addition of 92µl of 0.5%BSA/PBS and incubated for 20 to 60 min. Washing steps and incubation with secondary antibody are performed as described above for non-demasked serum.

### Results:

### Reactivity of mAbs with aggregates

To define whether aggregates allow the binding of proteinopathy-specific antibodies and to determine whether such an interaction can be monitored using the here described FACS-based assay another set of experiments is undertaken. For this purpose, aggregates as well as Aβ1-42 aggregates (made according to the disclosure in the international application PCT/EP2012/068494) are generated and are incubated with monoclonal antibodies specific either for aSyn (LB509) or Aβ1-42 (3A5). Additionally both forms of aggregates where incubated with the unspecific control mAB D129. As shown in FACS histograms in Figure 2, the monoclonal antibody LB509 binds exclusively aSyn aggregates whereas mAb 3A5 interacts only with Aβ1-42 aggregates. Furthermore, the irrelevant mAb D129 used as isotype control does neither react with aSyn nor with Aβ-aggregates. This shows that the described FACS-based assay allows the detection of aSyn antibodies in a highly specific manner.

### Defining the linear range of antibody reactivity against aSyn in FACS assay and ELISA system

To define the upper and lower limits of the linear analytic measurement range, titration experiments are performed using the mAb LB509 (Figure 3).

Calculations to estimate the linear range in Excel using the Pearson's coefficient of determination resulted in R2-values >0.9966 for LB509 concentrations in the range of 80-0.128ng/ml in the FACS aggregation assay, thereby going over three log stages. Determination of linearity in ELISA resulted in a linear range between 15.625 ng/ml and 0,489 ng/ml thereby going over only two log-stages. A R²-value of 1 would indicate 100% linearity.

### Reactivity of mAbs with mixed-aggregates

To define whether and to which extent mixed-aggregates allow the binding of aSyn, Aβ1-42, and p(E)Aβ3-42 specific antibodies and to determine whether such an interaction can be monitored using the here described FACS-based assay another set of experiments is undertaken. For this purpose, in addition to pure aggregates, Aβ1-42 aggregates and p(E)Aβ3-42 also equimolar mixtures or aggregate-mixtures with different ratios of individual proteins are generated as described in MM. These aggregates are then incubated with monoclonal antibodies specific either for aSyn (LB509) or Aβ1-42 (3A5) and p(E)Aβ3-42 (D129) and the mAB-binding pattern of the mixed aggregates is determined.

The reactivity of these mABs to three different aggregate substrates (established by mixing equimolar amounts of indicated peptides) is depicted in Figure 1. As shown in FACS PE-histograms, the monoclonal antibody 3A5 binds exclusively mixtures which contain Aβ1-42 aggregates (1A+B) whereas it shows no reactivity to the p(E)Aβ3-42/aSyn aggregate mixture (1C). The anti-p(E)Aβ3-42mAB D129 binds only mixtures that contain p(E)Aβ3-42 (1E+F) but shows no reactivity to Aβ1-42/aSyn (1D). Furthermore, the aSyn specific mAB LB509 reacts only with aggregate mixtures containing α-Synuclein (1G+I) but does not react with the Aβ1-42/p(E)Aβ3-42 aggregate mixture (1H). This shows first that mixed aggregates can be generated containing different peptides and second that these aggregates react specifically with the respective mAbs.

### Formation of mixed aggregates with differentially labelled peptides

As described in MM equimolar mixed aggregates with differentially fluorescent-labelled Aβ1-42 and α-Synuclein peptides are generated. Therefore, equimolar ratios of Aβ1-42-Hilyte-555 (detectable in the PE-channel) and α-Synuclein-Hilyte-488 (detectable in the FITC-channel) are mixed and aggregates are generated by overnight incubation. After incubation the mixed aggregates are analysed using the FACS Canto II. Acquired aggregates with a size range of 0.5-10 µm are gated in P1 in the FSC-A/SSC-A and P1 is further evaluated in the PE/FITC-dot plot to determine the contribution of Aβ1-42-Hilyte-555 (PE) and α-Synuclein-Hilyte-488 (FITC). Coincubation of Aβ1-42-Hilyte-555 and α-Synuclein-Hilyte-488 resulted in mixed aggregates which are ∼90% double positive indicating a homogenous distribution of Aβ1-42 and α-Synuclein within the aggregate population.

In a further experiment the reactivity of specific α-Aβ1-42- and α-Synuclein-mAB against mixed aggregates with different peptide constitutions is examined and the p(E)Aβ3-42 specific AB is used as negative control. Therefore a quantitative, symmetric titration series using monomeric Aβ1-42 and α-Synuclein is used to generate different mixed-aggregates-substrates before sample preparation.

As expected there is no reactivity of the unspecific control AB D129 and also no reactivity with the α-Synuclein specific AB LB509 when 100% Aβ1-42 aggregates are used as a substrate. But the titration series reveal that as soon as 0,1%/1% of α-Synuclein is mixed with 99,9/99% of Aβ1-42, it is sufficient to induce a strong reactivity for the α-Synuclein specific AB LB509 which was even stronger than the signals reached when LB509 is incubated with 100% α-Synuclein-aggregates. One reason for this could be that α-Synuclein alone forms aggregates with very tightly packed epitopes which inhibits the mAB to access all available epitopes because of sterical hindrance. On the other hand, in the mixed aggregates α-Synuclein is incorporated in way that all available epitopes can be accessed by the mAB without blocking itself and therefore the PE-MFI signal is increased compared to measurements when 100% α-Synuclein aggregates are used as a substrate (see also: Figs. 2 to 4).

### Example 2: Tau/Aβ1-42 aggregates

### Materials and Methods

### Phosphorylation of recombinant human Tau-441 protein with CDK5 and GSK3beta kinases

Recombinant human Tau - 441 protein (rPeptide) is phosphorylated with CDK5 and GSK3beta. For the sequential phosphorylation 100 µg of recombinant human Tau-441 protein is incubated with 1mM ATP, 1x reaction buffer and 2µg CDK5/p25 (Sigma-Aldrich) or 1000U of the kinase GSK3beta (New England BioLabs) at 30°C for 2h. In case of double phosphorylation, CDK5 incubation (as described above) is followed by incubation with 1000 U of the second kinase GSK3beta (New England BioLabs) and the mix is incubated at 30°C for further 16h. Phosphorylation of different serine and threonine residues is analyzed with Western Blot or ELISA assay.

### Production of Tau/hyperphosphorylated Tau aggregates for subsequent analyses

Lyophilized Tau is dissolved in sterile filtered H₂O by resuspending and short vortexing until solution is clear. Subsequently, this solution is sonicated for 30 sec in a sonication water bath and stored in aliquots at -20°C without shock-freezing.

To induce the formation of aggregates, aliquots are thawed and the entire volume is either subjected to direct aggregation or mixed with in vitro hyperphosphorylated Tau (ratios used range from 1 (Tau)/99(hyperphosphorylated Tau)-1(Tau)/1(hyperphosphorylated Tau)-99(Tau)/1(hyperphosphorylated Tau), respectively) and subsequently incubated for aggregation. The Tau/hyperphosphorylated Tau solution is incubated at a concentration of 10-100 µM in PBS or Hepes Buffer (pH7.4) at 37°C on shaker (350 rpm) overnight in an Eppendorf tube. Tau/hyperphosphorylated Tau aggregates are used for one experiment only and residuals are discarded.

### Reactivity of mAbs with mixed-aggregates of Tau/hyperphosphorylated Tau, Aβ1- and p(E)Aβ3-42

To define whether and to which extent mixed-aggregates allow the binding of Tau/hyperphosphorylated Tau, Aβ1-42, and p(E)Aβ3-42 specific antibodies and to determine whether such an interaction can be monitored using the here described FACS-based assay another set of experiments is undertaken. For this purpose, in addition to pure Tau/hyperphosphorylated Tau-aggregates, Aβ1-42 aggregates and p(E)Aβ3-42 also equimolar mixtures or aggregate-mixtures with different ratios of individual proteins are generated as described in Material and Methods (MM). These aggregates are then incubated with monoclonal antibodies specific either for Tau/hyperphosphorylated Tau (HT7) or Aβ1-42 (3A5) and p(E)Aβ3-42 (D129) and the mAB-binding pattern of the mixed aggregates is determined.

The reactivity of these mABs to three different aggregate substrates (established by mixing equimolar amounts of indicated peptides) can be shown in FACS PE-histograms, the monoclonal antibody 3A5 binds exclusively mixtures which contain Aβ1-42 aggregates whereas it shows no reactivity to the p(E)Aβ3-42/Tau/hyperphosphorylated Tau aggregate mixture. The anti-p(E) Aβ3-42mAB D129 binds only mixtures that contain p(E)Aβ3-42 but shows no reactivity to Aβ1-42/Tau/hyperphosphorylated Tau. Furthermore, the Tau/hyperphosphorylated Tau specific mAB HT7 reacts only with aggregate mixtures containing Tau/hyperphosphorylated Tau but does not react with the Aβ1-42/p(E)Aβ3-42 aggregate mixture. This indicates first that mixed aggregates can be generated containing different peptides and second that these aggregates react specifically with the respective mAbs.

In a further experiment the reactivity of specific α-Aβ1-42- and Tau/hyperphosphorylated Tau-mAB against mixed aggregates with different peptide constitutions is examined and the p(E)Aβ3-42 specific AB is used as negative control. Therefore a quantitative, symmetric titration series using monomeric Aβ1-42 and Tau/hyperphosphorylated Tau is used to generate different mixed-aggregates-substrates before sample preparation. In detail solutions of Aβ1-42 and Tau/hyperphosphorylated Tau (hpTau) are mixed in the ratios as shown in Table 2 and the PE-MFI is determined after sample preparation.

**Table 2**

| | | % | µl |
|---|---|---|---|
| Substrate 1: | Aβ1-42/hpTau | 100/0 | 100/0 |
| Substrate 2: | Aβ1-42/hpTau | 99.9/0.1 | 99.9/0.1 |
| Substrate 3: | Aβ1-42/hpTau | 99/1 | 99/1 |
| Substrate 4: | Aβ1-42/hpTau | 90/10 | 90/10 |
| Substrate 5: | Aβ1-42/hpTau | 50/50 | 50/50 |
| Substrate 6: | Aβ1-42/hpTau | 10/90 | 10/90 |
| Substrate 7: | Aβ1-42/hpTau | 1/99 | 1/99 |
| Substrate 8: | Aβ1-42/hpTau | 0.1/99.9 | 0.1/99.9 |
| Substrate 9: | Aβ1-42/hpTau | 0/100 | 0/100 |

There is no reactivity of the unspecific control AB D129 and also no reactivity with the Tau/hyperphosphorylated Tau specific AB HT7 when 100% Aβ1-42 aggregates are used as a substrate. But the titration series reveal that as soon as a small proportion of Tau/hyperphosphorylated Tau is mixed with Aβ1-42, it is sufficient to induce a strong reactivity for the Tau/hyperphosphorylated Tau specific AB HT7.

### Detection of Tau/hyperphosphorylated Tau-specific antibodies using ELISA

Human recombinant Tau-441 (purchased from rPeptide) is diluted in PBS (Gibco, neutral pH) at a concentration of 1µg/ml and coated on Maxisorp 96-well plates overnight. To prevent unspecific binding plates are blocked using 1% BSA/PBS at 37°C for 1h. The ELISA is performed with a serial dilution of mAbs (starting with indicated concentration) in binding buffer (PBS/0.1% BSA/0.1% Tween20) at 37°C for 1h. After repeated washing steps (3x) with PBS/0.1% Tween20 the secondary biotinylated anti-mouse/human/rabbit Ig (0.25µg/ml depending on host species of mAb/Ab used) antibody is added for 1h at 37°C. Samples are washed again 3x, followed by Strepatavidin-HRP (1h at 37°C, 0,1U/ml) incubation and 3 washing steps. Finally, ABTS (0.68 mM in 0.1 M citric acid pH 4.3) is added for 30 min for assay development before OD-measurement on plate reader (Biotek - Gen5 Program) at wave length 405 nm.

### Detection of Tau-specific antibodies using FACS analysis

The amount of Tau/hyperphosphorylated Tau aggregates (produced as described above) is determined to ensure comparable Tau/hyperphosphorylated Tau aggregate density before sample preparation for every experiment.

The number of aggregates generated after overnight incubation is measured in the FSC/SSC channel before sample preparation and the number of aggregates used as a substrate is normalized. In detail 3 µl of overnight generated Tau/hyperphosphorylated Tau-aggregates are diluted in 97 µl of 0.5% BSA/PBS as described above. 3µl represent the standard amount of substrate used for each sample. Using the FACS Canto II, 70 µl of this Tau/hyperphosphorylated Tau-dilution is measured at high flow rate (2µl/s) resulting in an acquisition time of 35 seconds and the number of aggregates is determined. Approximately 10.000 particles acquired under these conditions are defined as standard. If more or less aggregates are detected, the volume of Tau/hyperphosphorylated Tau-aggregate solution used as a substrate for further sample preparation is adjusted to ensure comparable aggregate density within samples in different experiments. This means that if for example only 5000 events are acquired the volume of substrate for sample preparation is increased to 6µl per well. As indicated above ∼10.000 particles are used as starting material for each sample preparation.

For the detection of Tau/hyperphosphorylated Tau-specific antibodies 3 µl of the Tau/hyperphosphorylated Tau-aggregate suspension are mixed with 92 µl of 0.2 µm filtered 0.5% BSA/PBS and subsequently transferred into a well of a 96-well V-shaped bottom plate for further sample preparation. These suspensions are incubated for 60 min at RT for blocking.

Aliquots of murine or human serum samples from -80°C are thawed freshly for each measurement, diluted 1:50 in case of murine serum samples or diluted 1:5 in case of human serum samples in 0.5% BSA/PBS and subsequently these samples are 0.2 µm filtered by centrifugation (3000 rpm for 3 min) through 0.22 µm 96well-filter plates in a 96-well plate-centrifuge. CSF samples are prediluted 1:5 in 0.5%BSA/PBS and are not sterile filtered. 5 µl of pre-diluted serum- or CSF- samples are added to 95 µl of the Tau/hyperphosphorylated Tau-aggregate-suspension resulting in final murine serum dilution of 1:1000 and a human serum or CSF dilution of 1:100. After 60 min incubation at RT on a shaker (450 rpm) 150 µl of 0.5% BSA/PBS is added to every well. The plate is centrifuged for 5 min at 3000 rpm (96-well plate-centrifuge) and the supernatant (SN) is removed by thoroughly pouring it into the sink. Thereafter, 200 µl of 0.5% BSA/PBS is added and this washing step is repeated 3 times. For the detection of murine serum and CSF samples after the 4th washing step the SN is discarded again and pellet is re-suspended in 100 µl 0.5% BSA/PBS containing a 1:10.000 dilution of anti-mIgG (H+L) F(ab')₂ Fragment labeled with PE (Jackson Immuno Research). For detection of IVIG a 1:1000 dilution of anti-hIgG (H+L) F(ab')₂ Fragment labeled with PE (Jackson Immuno Research) is used. Samples are incubated for another 60 min at RT on a shaker (600 rpm). Subsequently, samples are measured on a FACS Canto equipped with a high-throughput sampler (HTS) without an additional washing step. Tau/hyperphosphorylated Tau aggregates are identified based on their FSC/SSC characteristics. The signal of Abs binding to the aggregates is assessed in FL2-PE channel and is evaluated based on its median fluorescence intensity (MFI) using FACS Diva software. Generation and detection of co-aggregates and detection of specific antibody reactivity by FACS analysis using aggregates generated from mixed solutions of Tau/hyperphosphorylated Tau, Aβ1-42 and p(E)Aβ3-42 proteins as a substrate

100 µg of either Aβ1-42 or p(E)Aβ3-42 is dissolved in 100µl of sterile filtered 1%NH₄OH pH11 (Aβ1-42 and p(E)Aβ3-42) by resuspending and short vortexing until solution is clear. Subsequently these solutions are sonicated for 30 sec in a sonication water bath and stored in 5µl aliquots at -20°C without shock-freezing.

To induce the formation of co-aggregates with Tau/hyperphosphorylated Tau, aliquots from individual proteins are thawed and the entire volume of the three individual protein solutions are diluted to an equimolar concentration of ∼35 µM in 1% NH₄OH (pH4,5). These protein solutions are mixed in all three possible combinations before incubation at 37°C on shaker (350 rpm) for 20h in an Eppendorf tube as follows: Aβ1-42 & Tau/hyperphosphorylated Tau, Aβ1-42 & p(E)Aβ3-42 and p(E)Aβ3-42 & Tau/hyperphosphorylated Tau. Furthermore, not only equimolar ratios (=50:50) but also a titration experiment to test different ratios of aggregate mixtures is performed. The ratios of aggregate mixes are indicated in the experiment (e.g. 1:100=1 µl Tau/hyperphosphorylated Tau (20 µM) + 99 µl of Aβ1-42 (20 µM) solution). Mixed aggregates are used as substrates for further sample preparation and are treated as described for Tau/hyperphosphorylated Tau aggregates in paragraph above.

### Demasking

To disrupt the binding of Tau/hyperphosphorylated Tau specific autoantibodies to Tau/hyperphosphorylated Tau likely present in patient sera and, therefore, preventing detection of these Tau/hyperphosphorylated Tau bound auto-antibodies by antigen-based methods (like ELISA or FACS), sera are pre-diluted in 10mM Glycin pH2.6 at a dilution of 1:16.7 for 5min. 5µl of the acidified serum are then co-incubated with 3µl of Tau/hyperphosphorylated Tau for another 5min. Then the mixture is neutralized by addition of 92µl of 0.5%BSA/PBS and incubated for 20 to 60 min. Washing steps and incubation with secondary antibody are performed as described above for non-demasked serum.

### Results

In vitro phosphorylation of recombinant human Tau-441 with the candidate kinases GSK3beta and CDK5.

Hyperphosphorylated Tau has been described as one of the main pathologic hallmarks of Tauopathies like AD, PSP or CBD. A plethora of different cellular kinases have been implicated in playing a role in this physiologically and pathologically relevant process including PKN, CDK5, GSK3 (especially GSK3beta) MAPK and MARK. CDK5 and GSK3beta have been shown to be responsible in vitro to phosphorylate recombinant Tau at several sites resulting in the formation of stably hyperphosphorylated Tau protein. Therefore, recombinant human Tau-441 is phosphorylated as a prerequisite for Tau/hyperphosphorylated Tau aggregate formation using these two candidate kinases. Phosphorylation by either single kinases (GSK3beta and CDK5) or by both kinases consecutively is resulting in phosphorylation at the pathologically and diagnostically relevant residues as shown by ELISA: pT181 (used for CSF based diagnostics of AD in a commercially available ELISA system from Innogenetics; INNOTEST PHOSPHO TAU TM; www.innogenetics.com), pS202, pT205, pS212, pT214, pS396 and pT231 (see also: Fig 5). This result could also be reproduced partially by Western blot where pT181, pS202, pT205, pS396 and pT231 are detectable. Further potential S/T sites amenable to phosphorylation have not been tested but are likely to be phosphorylated as well using coincubation of both, CDK5 and GSK3beta (these residues comprise 153, 175, 199, 235, 262, 404, 422).

The discrepancy of ELISA and Western blot analysis is most probably due to differences in sensitivity of the two methods used.

### Tau/hyperphosphorylated Tau-aggregates: Oligomerization and fibril formation

The formation of Tau/hyperphosphorylated Tau-aggregates has been intensively investigated under multiple conditions in the recent years. It has been found that aggregation is very much dependent on different conditions including pH, temperature, buffer composition, protein concentration and the presence of aggregation promoters like polyanions or fatty acids and their derivatives. Aggregation starts from monomers leading to soluble oligomers. Conformational transition into parallel β-sheets then leads to the formation of fibrils and fibrillar aggregates, which can be precipitated by centrifugation.

According to the present invention, Tau/hyperphosphorylated Tau-aggregates are generated. These Tau/hyperphosphorylated Tau-aggregates can be detected using FACS-analysis. As described in MM for this purpose seedless soluble Tau/hyperphosphorylated Tau proteins are incubated for 20-48h at 37°C at a concentration of 10-100µM. A clear homogenous population of Tau/hyperphosphorylated Tau aggregates can be detected by FACS analysis. The size distribution of Tau/hyperphosphorylated Tau-aggregates (defined by forward scatter FSC-A) is analyzed using calibrated size beads ranging from 1 to 15 µm (Flow Cytometry Size Calibration Kit (Cat.# F-13838) by Molecular probes). Using this analysis it is shown that the size of generated Tau/hyperphosphorylated Tau-aggregates ranged as expected from sub-micrometer range up to 10 µm in which most of the generated aggregates range from ∼500 nm up to 2 µm.

### Reactivity of mAbs with Tau/hyperphosphorylated Tau-aggregates

To define whether Tau/hyperphosphorylated Tau-aggregates allow the binding of Tau/hyperphosphorylated Tau-specific antibodies and to determine whether such an interaction can be monitored using the here described FACS-based assay another set of experiments is undertaken. For this purpose, Tau/hyperphosphorylated Tau-aggregates as well as Aβ1-42 aggregates (made according to the disclosure in the international application PCT/EP2012/068494) are generated and are incubated with monoclonal antibodies specific either for Tau/hyperphosphorylated Tau (HT7) or Aβ1-42 (3A5). Additionally both forms of aggregates where incubated with the unspecific control mAB D129. The monoclonal antibody HT7 binds exclusively Tau/hyperphosphorylated Tau aggregates whereas mAb 3A5 interacts only with Aβ1-42 aggregates. Furthermore, the irrelevant mAb D129 used as isotype control does neither react with Tau/hyperphosphorylated Tau nor with Aβ-aggregates. This shows that the described FACS-based assay allows the detection of Tau/hyperphosphorylated Tau antibodies in a highly specific manner.

### Defining the linear range of antibody reactivity against Tau/hyperphosphorylated Tau in FACS assay and ELISA system

To define the upper and lower limits of the linear analytic measurement range, titration experiments are performed using the mAb HT7.

Calculations to estimate the linear range in Excel using the Pearson's coefficient of determination resulted in R2-values >0,9 for HT7 concentrations in a wide concentration range in the FACS aggregation assay, thereby going over three log stages. Determination of linearity in ELISA resulted in a linear range over only two log-stages. A R2-value of 1 would indicate 100% linearity.

### Defining the sensitivity of FACS aggregation assay and ELISA for Tau/hyperphosphorylated Tau binding of specific autoantibodies in sera and CSF of animals treated with Tau/hyperphosphorylated Tau specific vaccines

The aim of this experiment is to define and compare the detection limits of two independent detection methods (ELISA and the FACS-based assay) for Tau/hyperphosphorylated Tau-reactivity of sera and CSF samples derived from animals vaccinated with a Tau/hyperphosphorylated Tau specific vaccine. Tau/hyperphosphorylated Tau is therefore immobilized onto Maxisorp microtiter plates for ELISA measurements. Alternatively, Tau/hyperphosphorylated Tau aggregates are generated for FACS analysis. Subsequently, a dilution series of three different sera or a 1:100 dilution of 20 different murine CSF samples are applied to the individual systems and either OD at 405nm values in case of ELISA or fluorescence intensity (MFI values) in case of FACS assay is defined. For comparison reasons signal to noise ratio is evaluated for different serum- and CSF- measurements and signals are expressed as fold-background-signal (xBG). Higher sensitivity of the FACS aggregation assay is confirmed by titration of sera from immunized mice.

Furthermore, CSF samples from 20 mice are also analysed with both assays. Comparison of the two methods using a 1:100 dilution of the CSF samples results in only minor number of samples reaching a significant signal detection in ELISA whereas the majority out of 20 samples showed a MFI signal >2x BG when measured in the FACS aggregation assay.

This shows that the newly developed FACS-based assay to detect Tau/hyperphosphorylated Tau-specific autoantibodies is more sensitive than conventional assay systems such as ELISA.

### Defining the Tau/hyperphosphorylated Tau reactivity of human auto antibodies using IVIG

IVIG (intravenous immunoglobulin) is a commercially available blood product. It contains the pooled IgG fraction extracted from plasma derived from healthy donors (human plasma from at least 1000 donors). It has been shown that IVIG preparations contain naturally occurring antibodies (autoantibodies) specific for several pathogenic molecules including Aβ and aSyn-peptides (Dodel 2013).

The aim of this experiment is to define whether the here described technology offers the possibility to detect Tau/hyperphosphorylated Tau-specific autoantibodies in IVIG (IVIG-Subcuvia, purchased from Baxter, Austria) in an effective way.

For this purpose Tau/hyperphosphorylated Tau aggregates are generated for FACS analysis. Subsequently, different IVIG dilutions ranging from 200 µg - 64 ng/ml) are applied to the aggregates and fluorescence intensity (MFI values) are defined. The FACS-based assay provided strong fluorescence signals indicating that the newly developed FACS-based assay to detect Tau/hyperphosphorylated Tau-specific auto-antibodies is highly sensitive for Tau/hyperphosphorylated Tau autoantibodies.

### Defining anti- Tau/hyperphosphorylated Tau antibodies in human blood derived from healthy donors and TP patients a. Reactivity of IgG to Tau/hyperphosphorylated Tau

Serum samples derived either from healthy individuals or TP patients are analyzed for naturally occurring Tau/hyperphosphorylated Tau-specific antibody content using Tau/hyperphosphorylated Tau-aggregates and FACS analysis as described above. Naturally occurring antibodies specific for Tau/hyperphosphorylated Tau-aggregates are detected in all samples tested. However, the concentration of such antibodies is significantly different in blood samples derived from TP patients when compared to serum samples derived from healthy subjects.

To define the sensitivity and specificity of the present FACS assay to identify persons suffering from TP IgG reactivity to Tau/hyperphosphorylated Tau aggregates of sera are repeated twice and results are summarized in ROC curves. This ROC curve analysis shows very high specificity and sensitivity.

In an attempt to define a correlation between cognitive/functional and immunologic data the measured IgG reactivity to Tau/hyperphosphorylated Tau-aggregates of TP patient sera is correlated with scores from a functional assessment (e.g.: Mini-Mental State examination (MMSE)) of the patient's at the time of serum sampling. This correlation reveals that patients with weak test performance show a tendency to reduced IgG reactivity. This decrease of Tau/hyperphosphorylated Tau specific IgG reactivity reflects therefore the progression of TP.

In an attempt to define a correlation between cognitive/functional and immunologic data the measured IgG reactivity of AD patient sera is correlated with results from functional assessment (e.g.: Mini-Mental State examination (MMSE, a test which is commonly used to screen for cognitive impairment and dementia and estimates the severity of cognitive impairment in an individual at a given point in time). The correlation of MFI with assessment scores revealed that patients with weak test performance show a tendency to reduced IgG reactivity.

### b. Reactivity of IgM to different Tau/hyperphosphorylated Tau-aggregates

In a next set of experiments Tau/hyperphosphorylated Tau-aggregate specific IgM reactivity is defined in serum samples derived either from healthy individuals or TP patients, using the same sera as described above. Naturally occurring antibodies of IgM isotype specific for Tau/hyperphosphorylated Tau-aggregates are detected in all samples tested.

### c. Reactivity of IgG and IgM to Tau/hyperphosphorylated Tau-aggregates after demasking

To define whether Tau/hyperphosphorylated Tau antigens potentially bound to the auto-antibodies may block the reactivity of these antibodies to the in vitro generated Tau/hyperphosphorylated Tau-aggregates, the individual sera are subjected to a demasking procedure as described in material and methods. Using low pH potential immune complexes are disrupted resulting in removal of Tau/hyperphosphorylated Tau antigens from antibody binding domains (antigen dissociation). Thus a demasking procedure can result in higher autoantibody signals in the FACS based assay if immune complexes are present in serum samples.

ROC curve analysis shows high specificity and sensitivity for the assay following demasking.

To complete this set of experiments a demasking experiment is also performed to detect whether also autoantibodies of the IgG subtype might be occupied by Tau/hyperphosphorylated Tau antigens. For this purpose all sera are again treated with low pH to disrupt potential immune complexes and subsequently IgG reactivity of sera towards Tau/hyperphosphorylated Tau-aggregates is analyzed. As shown before in the IgM demasking experiment also in this experiment the IgG reactivity towards Tau/hyperphosphorylated Tau-aggregates of sera derived from healthy subjects did not differ between untreated and demasked sera. In contrast to this, sera derived from TP patients show again a significant change in Tau/hyperphosphorylated Tau-reactivity after the demasking procedure. Signals derived from binding of serum IgG autoantibodies to Tau/hyperphosphorylated Tau-aggregates before and after antigen dissociation are compared. The difference between the MFI values derived from the different measurements (untreated vs. demasked) are defined as the dissociation delta (Δ). Δ values of sera derived from healthy controls and Δ values of sera derived from AD patients are summarized in a ROC curve. As can be seen the Δ values derived from IgG reactivity can be used as well as parameter to identify TP patients.

The invention discloses the following preferred embodiments:
1. A method for detecting proteinopathy-specific antibodies in a biological sample comprising the following steps:
   - contacting the sample with aggregates, comprising two or more of a protein species selected from the group consisting of alpha synuclein (aSyn) or an aggregate-forming variant thereof, Tau protein or an aggregate-forming variant thereof, amyloid beta (Aβ)1-42 peptide or an aggregate-forming variant thereof; or contacting the sample with particles having aggregate like surfaces comprising two or more of said protein species, and allowing the proteinopathy-specific antibodies to bind to the aggregates, and
   - detecting the proteinopathy-specific antibodies bound to the aggregates by a single particle detection technique, preferably by fluorescence activated cell sorting (FACS).
2. A method according to embodiment 1, wherein the aggregate-forming aSyn variant, the aggregate-forming Tau protein variant, and the aggregate-forming Aβ1-42 variant is, independently of each other, selected from the following groups:
   (1) aSyn-126 (lacking aa 41-54; loss of exon 4), aSyn-112 (lacking aa 103-130; loss of exon 5), aSyn-98 (lacking exon 3 and 5), phosphorylated aSyn, aSyn-126 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids,
   (2) hyperphosphorylated Tau, abnormally phosphorylated Tau, Tau protein variants that have marker function for a disease, selected from either isoform of all 6 naturally occurring isoforms Tau441, Tau412, Tau410, Tau383, Tau381, and Tau352, mutant forms thereof, selected from P301L, P301S, V337M, Tau-variants simultaneously phosphorylated at amino acids 181, 202, 205, 212, 214, 231, 396 and, optionally, at one or more of the additional residues 18, 153, 175, 189, 235, 262, 394, 404 and 422 of Tau441, Tau412, Tau410, Tau383, Tau381, or Tau352; and
   (3) Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42; an Aβ variant selected from the group consisting of
      - a mutant of the Aβ-1-40- or the Aβ-1-42-peptide containing at least one mutation at an amino acid position other than amino acids 16 to 20, 25 to 28 and 31 to 35,
      - a truncated form of the Aβ-1-40- or the Aβ-1-42-peptide, wherein the truncation is at amino acid positions other than amino acids 16 to 35 and wherein the truncation is at least one amino acid and at most 20 amino acids; or
      - combinations thereof, especially combinations of at least one mutation and at least one truncation of the Aβ-1-40- or the Aβ-1-42-peptide.
3. Method according to embodiment 1 or 2, wherein the proteinopathy-specific antibodies are selected from the group consisting of anti-aSyn antibodies, anti-Tau antibodies, and anti-Aβ antibodies.
4. Method according to any one of embodiments 1 to 3, wherein the proteinopathy-specific antibodies are human antibodies, preferably human IgG or IgM antibodies, especially human IgG antibodies.
5. Method according to any one of embodiments 1 to 4, wherein the proteinopathy-specific antibodies are autoantibodies.
6. Method according to any one of embodiments 1 to 5, wherein the aggregates have a size of 50 nm to 15 µm, preferably from 100 nm to 10 µm, especially from 200 nm to 5 µm.
7. Method according to any one of embodiments 1 to 6, wherein the aggregates have been prepared by incubating the protein species at a pH of 2 to 9 for at least 20 min, preferably at least 1 h, especially at least 4 h.
8. Method according to any one of embodiments 1 to 7, wherein the aggregates are present for contacting the sample with aggregates in an amount of 0.01 to 10 µM, preferably 0.1 to 1 µM.
9. Method according to any one of embodiments 1 to 8, wherein the biological sample is human blood or a sample derived from human blood, preferably human serum or human plasma; human cerebrospinal fluid or human lymph.
10. Method according to any one of embodiments 1 to 9, wherein the aggregates are contacted with the sample for at least 10 min, preferably from 10 min to 24 h, especially from 20 min to 2 h.
11. Method according to any one of embodiments 1 to 10, wherein a demasking step is performed on the proteinopathy-specific antibodies in the sample before contacting the sample with the aggregates, especially if IgM antibodies are detected.
12. Method according to any one of embodiments 1 to 11, wherein the particles having aggregate like surfaces are beads with aggregates immobilised on their surface, especially magnetic beads.
13. Method according to any one of embodiments 1 to 12, wherein the biological sample is a sample of a patient undergoing or supposed to undergo aproteinopathy immunotherapy and wherein the detected amount of proteinopathy-specific antibodies is correlated to a proteinopathy-related diagnosis result of the same patient at the same time the sample was taken from the patient.
14. Method according to any one of embodiments 1 to 13, wherein the method is performed at least twice on samples of the same patient taken at a different time and wherein preferably the detected amounts of proteinopathy-specific antibodies is correlated to proteinopathy-related diagnosis results of the same patient at the same times the samples were taken from the patient.
15. Method according to embodiment 14, wherein the method is performed at least once each six months, preferably at least once each three months, especially at least once each month.
16. Method according to any one of embodiments 1 to 15, wherein the aggregate further comprises islet amyloid polypeptide (IAPP) and an aggregate-forming variant thereof, especially IAPP-Npro, TAR-DNA binding protein 43 (TDP43) or an aggregate-forming variant thereof; Superoxide-Dismutase 1 (SOD1) or an aggregate-forming variant thereof.
17. Use of a method according to any one of embodiments 1 to 16 for the diagnosis of proteinopathies, especially synucleinopathies, wherein the proteinopathy is preferably selected from Parkinson's disease (PD), Lewy Body Disease (LBD), Dementia with Lewy Bodies (DLB), Parkinson's Disease Dementia (PDD), Multiple System Atrophy (MSA), Pure Autonomic Failure (PAF), REM Sleep Behaviour Disorder (RBD), Neurodegeneration with Brain Iron Accumulation type I (NBIA Type I), inclusion body myositis (IBM), Cortico-Basal Degeneration (CBD), Progressive Supranuclear Palsy (PSP), Pick's Disease (PiD), Dementia pugilistica (chronic traumatic encephalopathy, DP), Frontotemporal dementia (FTD), Lytico-Bodig disease (LD), Huntington's disease (HD) and Spinocerebellar ataxias (Type 1, 2, 3 and 7) and Amyotrophic lateral sclerosis (ALS), Progressive Supranuclear Palsy (PSP), Corticobasal degeneration (CBD), Frontotemporal Dementia /Pick's Disease (FTD/PiD), prionosis and type II diabetes; especially for tracking early stages of the proteinopathy and for observing the development of clinical trials for drugs for the treatment of proteinopathies.
18. Kit for performing the method according to any one of embodiments 1 to 16 comprising
   - aggregates, as defined by anyone of embodiments 1 to 16, and
   - a sample container.
19. Aggregate, comprising two or more of a protein species selected from the group consisting of alpha synuclein (aSyn) or an aggregate-forming variant thereof, Tau protein or an aggregate-forming variant thereof, Aβ1-42 peptide or an aggregate-forming variant thereof.
20. Aggregate according to embodiment 19, wherein the aggregate-forming aSyn variant, the aggregate-forming Tau protein variant, the aggregate-forming Aβ1-42 variant and the aggregate-forming IAPP variant is, independently of each other, selected from the following groups:
   (1) aSyn-126 (lacking aa 41-54; loss of exon 4), aSyn-112 (lacking aa 103-130; loss of exon 5), aSyn-98 (lacking exon 3 and 5), phosphorylated aSyn, aSyn-126 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids,
   (2) hyperphosphorylated Tau, abnormally phosphorylated Tau, Tau protein variants that have marker function for a disease, selected from either isoform of all 6 naturally occurring isoforms Tau441, Tau412, Tau410, Tau383, Tau381, and Tau352, mutant forms thereof, selected from P301L, P301S, V337M, Tau-variants simultaneously phosphorylated at amino acids 181, 202, 205, 212, 214, 231, 396 and, optionally, at one or more of the additional residues 18, 153, 175, 189, 235, 262, 394, 404 and 422 of Tau441, Tau412, Tau410, Tau383, Tau381, or Tau352; and
   (3) Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42; an Aβ variant selected from the group consisting of
      - a mutant of the Aβ-1-40- or the Aβ-1-42-peptide containing at least one mutation at an amino acid position other than amino acids 16 to 20, 25 to 28 and 31 to 35,
      - a truncated form of the Aβ-1-40- or the Aβ-1-42-peptide, wherein the truncation is at amino acid positions other than amino acids 16 to 35 and wherein the truncation is at least one amino acid and at most 20 amino acids; or
      - combinations thereof, especially combinations of at least one mutation and at least one truncation of the Aβ-1-40- or the Aβ-1-42-peptide.
21. Aggregate according to embodiment 19 or 20, wherein the aggregate has a size of 50 nm to 15 µm, preferably from 100 nm to 10 µm, especially from 200 nm to 5 µm.
22. Aggregate according to any one of embodiments 19 to 21, wherein the aggregate is obtainable by incubating the protein species at a pH of 2 to 9 for at least 20 min, preferably at least 1 h, especially at least 4 h.
23. Aggregate according to any one of embodiments 19 to 22, wherein the aggregate comprises three or more, preferably four or more, especially five or more, of a protein species selected from the group consisting of alpha synuclein (aSyn) or an aggregate-forming variant thereof, Tau protein or an aggregate-forming variant thereof, Aβ1-42 peptide or an aggregate-forming variant thereof.
24. Aggregate according to any one of embodiments 19 to 23, wherein the aggregate comprises at least two protein species from different groups of the following groups (a) to (d):
   (a) alpha synuclein (aSyn) or an aggregate-forming variant thereof,
   (b) Tau protein or an aggregate-forming variant thereof,
   (c) Aβ1-42 peptide or an aggregate-forming variant thereof,
   (d) islet amyloid polypeptide (IAPP) or an aggregate-forming variant thereof, especially IAPP-Npro, TAR-DNA binding protein 43 (TDP43) or an aggregate-forming variant thereof; Superoxide-Dismutase 1 (SOD1) or an aggregate-forming variant thereof.
25. Aggregate according to any one of embodiments 19 to 24, wherein the aggregate comprises
   - at least one protein species from group (a): alpha synuclein (aSyn) or an aggregate-forming variant thereof; and at least one protein species from group (b): Tau protein or an aggregate-forming variant thereof, or
   - at least one protein species from group (a): alpha synuclein (aSyn) or an aggregate-forming variant thereof; and at least one protein species from group (c) Aβ1-42 peptide or an aggregate-forming variant thereof, or
   - at least one protein species from group (b): Tau protein or an aggregate-forming variant thereof; and at least one protein species from group (c) Aβ1-42 peptide or an aggregate-forming variant thereof.
26. Aggregate according to any one of embodiments 19 to 25, wherein the aggregate comprises aSyn and Aβ1-42 peptide, aSyn and hyperphosphorylated Tau protein, hyperphosphorylated Tau protein and Aβ1-42 peptide, or aSyn and Aβ1-42 peptide and hyperphosphorylated Tau protein.

## Claims

1. A method for detecting proteinopathy-specific antibodies in a biological sample comprising the following steps:
- contacting the sample with aggregates, comprising two or more of a protein species selected from the group consisting of alpha synuclein (aSyn) or an aggregate-forming variant thereof, Tau protein or an aggregate-forming variant thereof, amyloid beta (Aβ)1-42 peptide or an aggregate-forming variant thereof; or contacting the sample with particles having aggregate like surfaces comprising two or more of said protein species, and allowing the proteinopathy-specific antibodies to bind to the aggregates, and
- detecting the proteinopathy-specific antibodies bound to the aggregates by a single particle detection technique, preferably by fluorescence activated cell sorting (FACS).

2. A method according to claim 1, wherein the aggregate-forming aSyn variant, the aggregate-forming Tau protein variant, and the aggregate-forming Aβ1-42 variant is, independently of each other, selected from the following groups:
(1) aSyn-126 (lacking aa 41-54; loss of exon 4), aSyn-112 (lacking aa 103-130; loss of exon 5), aSyn-98 (lacking exon 3 and 5), phosphorylated aSyn, aSyn-126 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids,
(2) hyperphosphorylated Tau, abnormally phosphorylated Tau, Tau protein variants that have marker function for a disease, selected from either isoform of all 6 naturally occurring isoforms Tau441, Tau412, Tau410, Tau383, Tau381, and Tau352, mutant forms thereof, selected from P301L, P301S, V337M, Tau-variants simultaneously phosphorylated at amino acids 181, 202, 205, 212, 214, 231, 396 and, optionally, at one or more of the additional residues 18, 153, 175, 189, 235, 262, 394, 404 and 422 of Tau441, Tau412, Tau410, Tau383, Tau381, or Tau352; and
(3) Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42; an Aβ variant selected from the group consisting of
- a mutant of the Aβ-1-40- or the Aβ-1-42-peptide containing at least one mutation at an amino acid position other than amino acids 16 to 20, 25 to 28 and 31 to 35,
- a truncated form of the Aβ-1-40- or the Aβ-1-42-peptide, wherein the truncation is at amino acid positions other than amino acids 16 to 35 and wherein the truncation is at least one amino acid and at most 20 amino acids; or
- combinations thereof, especially combinations of at least one mutation and at least one truncation of the Aβ-1-40- or the Aβ-1-42-peptide.

3. Method according to claim 1 or 2, wherein the proteinopathy-specific antibodies are selected from the group consisting of anti-aSyn antibodies, anti-Tau antibodies, and anti-Aβ antibodies.

4. Method according to any one of claims 1 to 3, wherein the proteinopathy-specific antibodies are human antibodies, preferably human IgG or IgM antibodies, especially human IgG antibodies.

5. Method according to any one of claims 1 to 4, wherein the proteinopathy-specific antibodies are autoantibodies.

6. Method according to any one of claims 1 to 5, wherein the aggregates have a size of 50 nm to 15 µm, preferably from 100 nm to 10 µm, especially from 200 nm to 5 µm.

7. Use of a method according to any one of claims 1 to 6 for the diagnosis of proteinopathies, especially synucleinopathies, wherein the proteinopathy is preferably selected from Parkinson's disease (PD), Lewy Body Disease (LBD), Dementia with Lewy Bodies (DLB), Parkinson's Disease Dementia (PDD), Multiple System Atrophy (MSA), Pure Autonomic Failure (PAF), REM Sleep Behaviour Disorder (RBD), Neurodegeneration with Brain Iron Accumulation type I (NBIA Type I), inclusion body myositis (IBM), Cortico-Basal Degeneration (CBD), Progressive Supranuclear Palsy (PSP), Pick's Disease (PiD), Dementia pugilistica (chronic traumatic encephalopathy, DP), Frontotemporal dementia (FTD), Lytico-Bodig disease (LD), Huntington's disease (HD) and Spinocerebellar ataxias (Type 1, 2, 3 and 7) and Amyotrophic lateral sclerosis (ALS), Progressive Supranuclear Palsy (PSP), Corticobasal degeneration (CBD), Frontotemporal Dementia /Pick's Disease (FTD/PiD), prionosis and type II diabetes; especially for tracking early stages of the proteinopathy and for observing the development of clinical trials for drugs for the treatment of proteinopathies.

8. Kit for performing the method according to any one of claims 1 to 6 comprising
- aggregates, as defined by anyone of claims 1 to 6, and
- a sample container.

9. Aggregate, comprising two or more of a protein species selected from the group consisting of alpha synuclein (aSyn) or an aggregate-forming variant thereof, Tau protein or an aggregate-forming variant thereof, Aβ1-42 peptide or an aggregate-forming variant thereof.

10. Aggregate according to claim 9, wherein the aggregate-forming aSyn variant, the aggregate-forming Tau protein variant, the aggregate-forming Aβ1-42 variant and the aggregate-forming IAPP variant is, independently of each other, selected from the following groups:
(1) aSyn-126 (lacking aa 41-54; loss of exon 4), aSyn-112 (lacking aa 103-130; loss of exon 5), aSyn-98 (lacking exon 3 and 5), phosphorylated aSyn, aSyn-126 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids,
(2) hyperphosphorylated Tau, abnormally phosphorylated Tau, Tau protein variants that have marker function for a disease, selected from either isoform of all 6 naturally occurring isoforms Tau441, Tau412, Tau410, Tau383, Tau381, and Tau352, mutant forms thereof, selected from P301L, P301S, V337M, Tau-variants simultaneously phosphorylated at amino acids 181, 202, 205, 212, 214, 231, 396 and, optionally, at one or more of the additional residues 18, 153, 175, 189, 235, 262, 394, 404 and 422 of Tau441, Tau412, Tau410, Tau383, Tau381, or Tau352; and
(3) Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42; an Aβ variant selected from the group consisting of
- a mutant of the Aβ-1-40- or the Aβ-1-42-peptide containing at least one mutation at an amino acid position other than amino acids 16 to 20, 25 to 28 and 31 to 35,
- a truncated form of the Aβ-1-40- or the Aβ-1-42-peptide, wherein the truncation is at amino acid positions other than amino acids 16 to 35 and wherein the truncation is at least one amino acid and at most 20 amino acids; or
- combinations thereof, especially combinations of at least one mutation and at least one truncation of the Aβ-1-40- or the Aβ-1-42-peptide.

11. Aggregate according to claim 9 or 10, wherein the aggregate has a size of 50 nm to 15 µm, preferably from 100 nm to 10 µm, especially from 200 nm to 5 µm.

12. Aggregate according to any one of claims 9 to 11, wherein the aggregate is obtainable by incubating the protein species at a pH of 2 to 9 for at least 20 min, preferably at least 1 h, especially at least 4 h.

13. Aggregate according to any one of claims 9 to 12, wherein the aggregate comprises three or more, preferably four or more, especially five or more, of a protein species selected from the group consisting of alpha synuclein (aSyn) or an aggregate-forming variant thereof, Tau protein or an aggregate-forming variant thereof, Aβ1-42 peptide or an aggregate-forming variant thereof.

14. Aggregate according to any one of claims 9 to 13, wherein the aggregate comprises at least two protein species from different groups of the following groups (a) to (d):
(a) alpha synuclein (aSyn) or an aggregate-forming variant thereof,
(b) Tau protein or an aggregate-forming variant thereof,
(c) Aβ1-42 peptide or an aggregate-forming variant thereof,
(d) islet amyloid polypeptide (IAPP) or an aggregate-forming variant thereof, especially IAPP-Npro, TAR-DNA binding protein 43 (TDP43) or an aggregate-forming variant thereof; Superoxide-Dismutase 1 (SOD1) or an aggregate-forming variant thereof.

15. Aggregate according to any one of claims 9 to 14, wherein the aggregate comprises
- at least one protein species from group (a): alpha synuclein (aSyn) or an aggregate-forming variant thereof; and at least one protein species from group (b): Tau protein or an aggregate-forming variant thereof, or
- at least one protein species from group (a): alpha synuclein (aSyn) or an aggregate-forming variant thereof; and at least one protein species from group (c) Aβ1-42 peptide or an aggregate-forming variant thereof, or
- at least one protein species from group (b): Tau protein or an aggregate-forming variant thereof; and at least one protein species from group (c) Aβ1-42 peptide or an aggregate-forming variant thereof.
